Europäisches Patentamt

European Patent Office

Office européen des brevets

(11) Veröffentlichungsnummer: **0 232 523 B1**

(12) **EUROPÄISCHE PATENTSCHRIFT**

(45) Veröffentlichungstag der Patentschrift: **21.04.93**

(21) Anmeldenummer: **86117504.0**

(22) Anmeldetag: **16.12.86**

(51) Int. Cl.5: **C12N 15/12**, C12N 1/21, C07K 7/10, C12P 21/02, A61K 37/64, A61K 37/02

(54) DNA-Sequenzen, die für Proteine mit der biologischen Aktivität der HUSI-TypI-Inhibitoren codieren, gentechnologische Verfahren zur Herstellung dieser Proteine und diese Proteine enthaltende Arzneimittel.

(30) Priorität: **10.01.86 DE 3600571**

(43) Veröffentlichungstag der Anmeldung:
**19.08.87 Patentblatt 87/34**

(45) Bekanntmachung des Hinweises auf die
Patenterteilung:
**21.04.93 Patentblatt 93/16**

(84) Benannte Vertragsstaaten:
**AT BE CH DE ES FR GB GR IT LI LU NL SE**

(56) Entgegenhaltungen:
**WO-A-86/03519**

**FEBS, Band 199, Nr. 1, April 1986, Seiten 43-48; Amsterdam, NL-U. SEEMUELLER et al. :"The axid-stable proteinase inhibitor of human mucous secretions (HUSI-I, antileukoprotease)"**

(73) Patentinhaber: **Grünenthal GmbH**
**Zieglerstrasse 6**
**W-5100 Aachen(DE)**

(72) Erfinder: **Heinzel, Regina**
**Kroitzheiderweg 50**
**W-5100 Aachen(DE)**
Erfinder: **Appelhans, Heribert, Dr.**
**Am Bordenbergweg 17**
**W-6109 Trautheim(DE)**
Erfinder: **Gassen, Hans Günther, Prof. Dr.**
**Siemensstrasse 8**
**W-6100 Darmstadt(DE)**
Erfinder: **Machleidt, Werner, Prof. Dr.**
**Koboldstrasse 63**
**W-8000 München 83(DE)**
Erfinder: **Seemüller, Ursula, Dr.**
**Stumpengartenweg 14**
**W-7290 Freudenstadt(DE)**

(74) Vertreter: **Vossius & Partner**
**Siebertstrasse 4 P.O. Box 86 07 67**
**W-8000 München 86 (DE)**

EUROPEAN JOURNAL OF BIOCHEMISTRY, Band 160, NR.1, September 1986, Seiten 61-67 ; Berlin, DE - R. HEINZEK et al.:"Molecular cloning and expression of cDNA for human antileukoprotease from cervix uterus"

NEUTRAL PROTEASES OF HUMAN POLY-MORPHONUCLEAR LEUKOCYTES, 1978, pp. 195-207 ; Urban & Schwarzenberg, Inc. Baltimore, US - H. SCHIESSLER et al.:" Acid-stable inhibitors of granulocyte neutral proteases in human mucous secretions : biochemistry and possible biological function"

PROTEIN DEGRADATION IN HEALTH AND DISEASE, Ciba Foundation Symposium 75(new series), 1980, Seiten 351-379, Excerpta Medica, Amsterdam, NL - H.FRITZ :"Proteinase inhibitors in severe inflammatory processes (septic shock and experimental endotoxaemia): biochemical, pathophysiological and therapeutic aspects"

BIOCHEMICAL AND BIOPHYSICAL RESEARCG COMMUNICATIONS, Band 128, Nr. 1, 16 April 1985, Seiten 285-289, Acad. Press, Inc., New York, US - E.C. KLASEN et al.:"The N-terminal sequence of antileukoprotease isolated from bronchial secretion"

AGENTS AND ACTIONS, Band 8, Nr. 1,2, 1978, Seiten 57-64 ; Basel, CH - H.FRITZ et al.:"Naturally occuring low molecular weight inhibitors of neutral proteinases from PMN-granulocytes and of kallikreins"

## Beschreibung

Die Erfindung betrifft DNA-Sequenzen aus dem Genom von Säugern, insbesondere aus dem des Menschen, die für Proteine mit der biologischen Aktivität der HUSI-TypI-Inhibitoren codieren, sowie Clonierungs- und Expressions-Vektoren, die solche DNA-Sequenzen enthalten.

Ferner betrifft die Erfindung mit den genannten Vektoren transformierte Wirtsorganismen und gentechnologische Verfahren zur Herstellung von Proteinen mit der biologischen Aktivität der HUSI-TypI-Inhibitoren unter Verwendung der transformierten Wirtsorganismen.

Schließlich betrifft die Erfindung Proteine mit der biologischen Aktivität der HUSI-TypI-Inhibitoren sowie solche Proteine enthaltende Arzneimittel.

In lebenden Zellen und Organismen wird die Aktivität von Enzymen zunächst durch die Neusynthese und die chemische Modifikation von Enzymen reguliert.

Für eine schnelle Anpassung einer Zelle oder eines Organismus an eine veränderte Umweltsituation, die eine erhöhte Aktivität eines bestimmten Enzyms erfordert, findet jedoch nicht in jedem Fall eine erhöhte Neusynthese dieses Enzyms statt. Häufig wird ein bereits bestehender Enzymvorrat aktiviert.

Beispielsweise werden Verdauungsenzyme (Proteinasen) von der Vorratsform, den sogenannten Zymogenen, zur aktiven Proteinase überführt. Ebenso werden Blutgerinnungsfaktoren im Bedarfsfall von der inaktiven Vorratsform in die biologisch aktive Form überführt.

Als Aktivierungsmechanismen von Vorratsenzymen sind die Spaltung durch spezifische Peptidasen, die Phosphorylierung durch Proteinkinasen, die Ausschüttung aus Vesikeln und die Änderung der Proteinkonformation durch allosterische Liganden bekannt.

Ein Überschießen der genannten Aktivierungsreaktionen und die Langzeitwirkung der aktivierten Enzyme wird durch den gezielten Abbau oder die spezifische Inhibition dieser Enzyme vermieden.

Beispielsweise wird die biologische Aktivität aktivierter Proteinasen häufig durch spezifische Proteinase-Inhibitoren blockiert.

In den letzten Jahren wurde die klinische und pathogenetische Relevanz verschiedener Proteinase-Inhibitoren erkannt (1, 2).

Es wurde gefunden, daß lysosomale Proteinase-Inhibitoren zur Therapie von Sepsen, von chronischen Erkrankungen des rheumatischen Formenkreises sowie von Erkrankungen des oberen Lungentraktes geeignet sind.

Es sind jedoch im Augenblick keine bei diesen Erkrankungen therapeutisch einsetzbaren Proteinase-Inhibitoren bekannt. Derzeit wird lediglich der Proteinase-Inhibitor Aprotinin therapeutisch angewendet. Aprotinin wird zur Behandlung postoperativer Blutungen durch Hyperfibrinolysen und zur Frühtherapie von Schockzuständen eingesetzt.

Die HUSI (Human-Seminalplasma-Inhibitor)-TypI-Inhibitoren, bei denen es sich um Proteine handelt, könnten für die Therapie der vorstehend genannten Erkrankungen geeignet sein. Zur Gruppe der HUSI-TypI-Inhibitoren gehören die Proteinase-Inhibitoren HUSI-I, CUSI-I (Cervix-Uterus-Sekret-Inhibitor) und BSI (Bronchial-Sekret-Inhibitor).

HUSI-I ist ein säurestabiler Proteinaseinhibitor aus dem menschlichen Seminalplasma, der Proteinasen aus den lysosomalen Granula der Granulozyten inhibiert, beispielsweise die Elastase. Gegen andere intra- und extrazelluläre Proteinasen zeigt HUSI-I nur eine abgeschwächte Hemmaktivität. Sein Molekulargewicht beträgt etwa 11 000. Eine Aminosäureteilsequenz von HUSI-I wurde von Fritz (48) veröffentlicht.

Neben HUSI-I gibt es im menschlichen Seminalplasma noch den weiteren säurestabilen Proteinase-Inhibitor HUSI-II, dessen Molekulargewicht ca. 6500 beträgt (3).

HUSI-I und HUSI-II besitzen völlig unterschiedliche Hemmspektren. Während die Hemmaktivität von HUSI-II auf Trypsin und Akrosin beschränkt bleibt, ist die bemerkenswerteste Eigenschaft des HUSI-I die spezifische Inaktivierung von Proteasen aus den lysosomalen Granula der Granulozyten, beispielsweise der Elastase. Aufgrund seiner abweichenden biologischen Aktivität ist somit HUSI-II kein HUSI-TypI-Inhibitor.

Der säurestabile Inhibitor CUSI-I wurde aus dem Cervix-Uterus-Sekret isoliert (4).

Das Molekulargewicht von CUSI-I ist nahezu identisch mit dem von HUSI-I. HUSI-I und CUSI-I besitzen ferner das gleiche Hemmspektrum. Im Ouchterlony-Immundiffusionstest zeigen HUSI-I und CUSI-I immunologische Kreuzreaktion mit anti-HUSI-I-Antikörpern(5, 6). Schließlich sind die bislang nur lückenhaft bekannten Aminosäureanalysen von HUSI-I und CUSI-I nahezu identisch (47).

Der Bronchialsekret-Inhibitor (BSI) wurde aus dem Bronchialsekret isoliert (41, 44, 45, 46). Die Sequenz der ersten 25 Aminosäuren von BSI wurde unvollständig veröffentlicht in (41). BSI hat ein Molekulargewicht von etwa 10 ooo. In immunologischen Tests zeigt BSI eine Kreuzreaktion mit anti-HUSI-I-Antikörpern aus Kaninchen (47). BSI ist säurestabil und inhibiert die Proteinasen Leukozytenelastase, Cathepsin G, Trypsin und Chymotrypsin.

Obwohl somit die biologische Aktivität der HUSI-TypI-Inhibitoren im wesentlichen bekannt war, konnten diese Inhibitoren bislang nicht in der Therapie eingesetzt werden, da sie nicht in ausreichender Menge und in reiner Form zur Verfügung standen.

Somit liegt der vorliegenden Erfindung die Aufgabe zugrunde, DNA-Sequenzen bereitzustellen, die für Proteine mit der biologischen Aktivität der HUSI-TypI-Inhibitoren codieren und unter Verwendung solcher DNA-Sequenzen Proteine mit der biologischen Aktivität der HUSI-TypI-Inhibitoren mit Hilfe gentechnologischer Verfahren herzustellen.

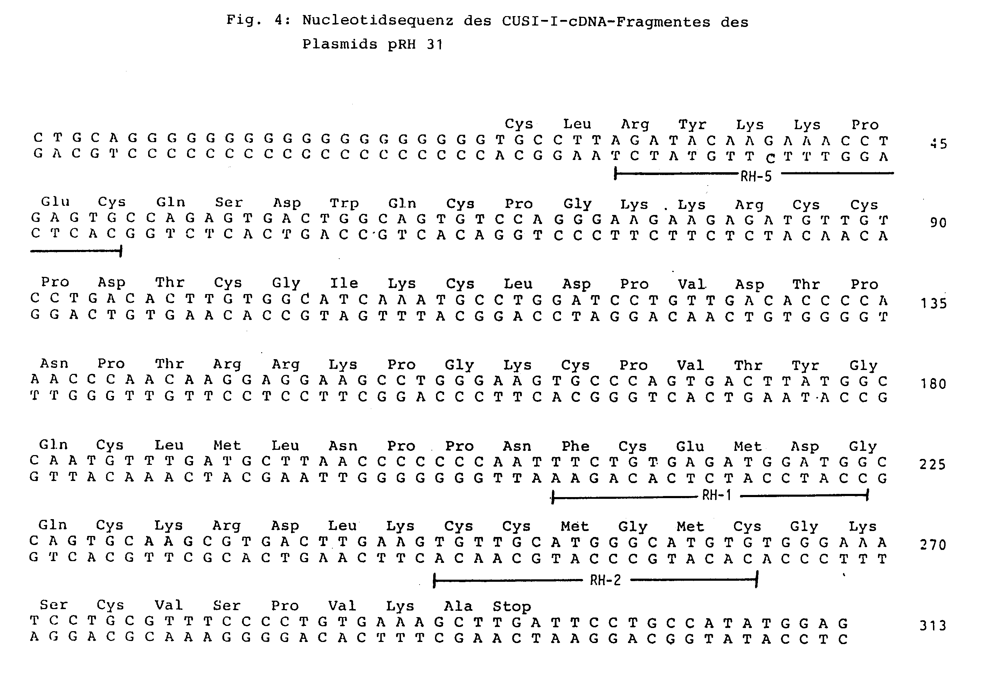

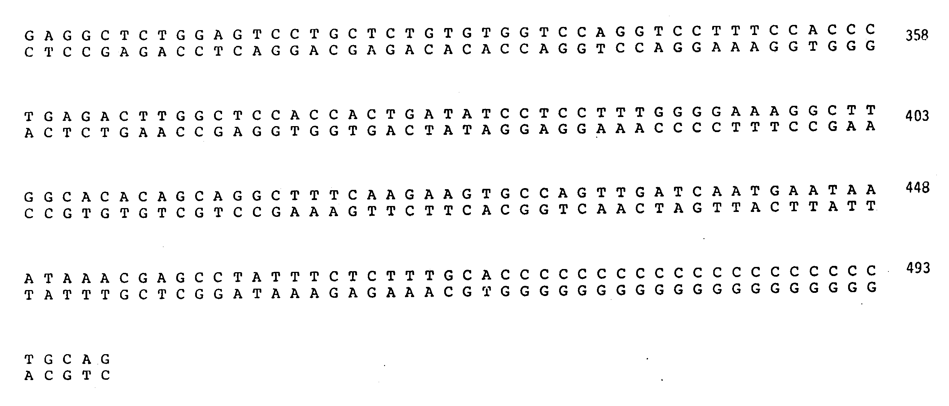

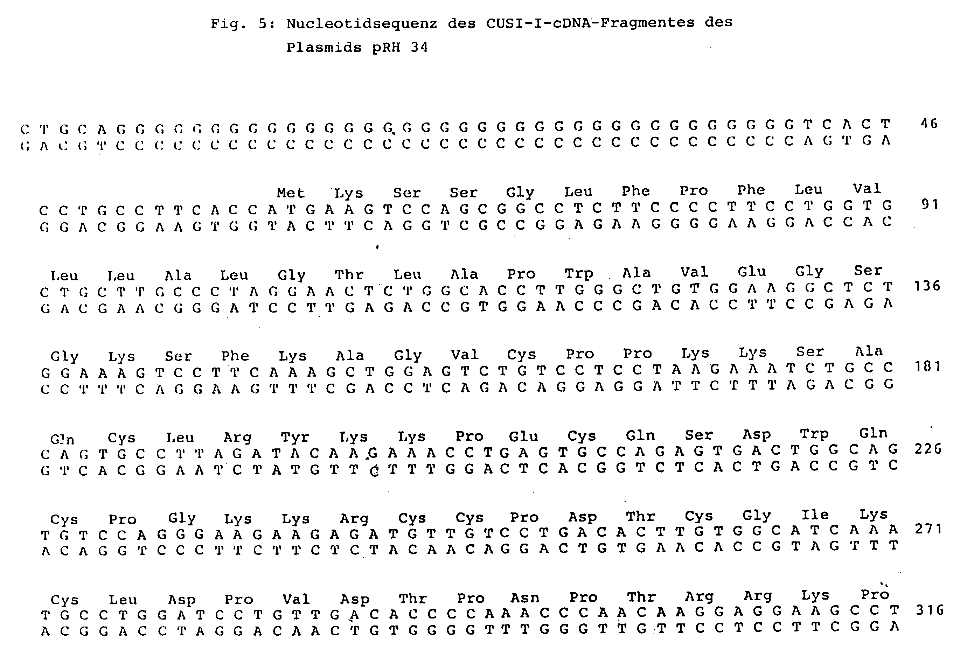

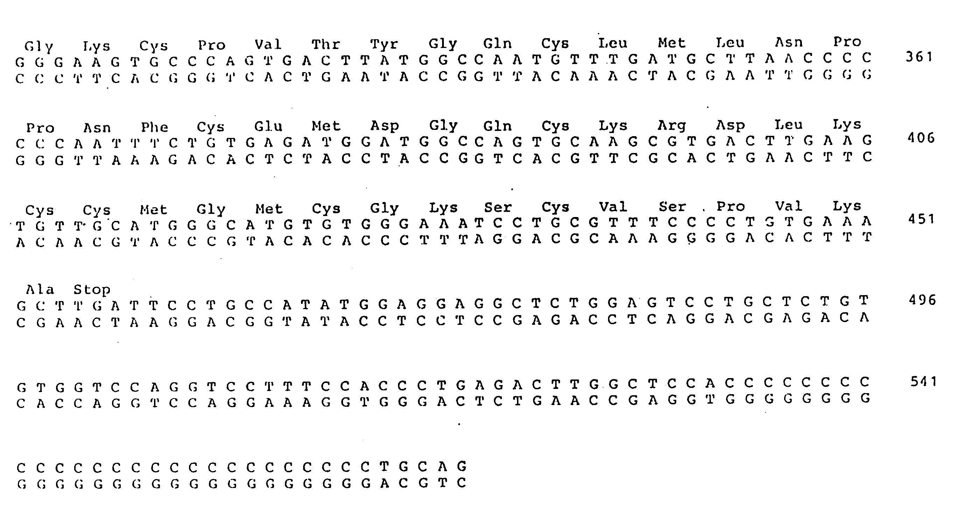

Diese Aufgabe wird durch die Bereitstellung von DNA-Sequenzen gelöst, die aus dem Genom eines Säugers stammen, insbesondere aus dem menschlichen Genom, die ein Protein mit der biologischen Aktivität des CUSI-I-Proteins mit der in Figur 4 oder 5 dargestellten Aminosäuresequenz codiert.

Erfindungsgemäß bezieht sich die Bezeichnung "Proteine mit der biologischen Aktivität der HUSI-TypI-Inhibitoren" auf Fusionsproteine und Nichtfusionsproteine, die die biologische Aktivität des Inhibitors CUSI-I aufweisen, also beispielsweise über immunologische Eigenschaften der natürlichen Proteine verfügen und/oder die spezifischen inhibitorischen Eigenschaften der natürlichen Proteine haben. Die inhibitorische Aktivität der erfindungsgemäßen Proteine mit der biologischen Aktivität des CUSI-I-Inhibitors wird nachstehend durch die Messung der Hemmung des Enzyms Chymotrypsin ermittelt.

Auch Fusionsproteine und Nichtfusionsproteine, die nur Teilbereiche der Aminosäuresequenzen von CUSI-I umfassen, werden erfindungsgemäß als Proteine mit der biologischen Aktivität des CUSI-I-Inhibitors bezeichnet. Teilbereiche der Aminosäuresequenz von Proteinen werden auch Domänen genannt.

In einer bevorzugten Ausführungsform der vorliegenden Erfindung codiert die DNA-Sequenz für ein Protein mit der in Figur 5 dargestellten Aminosäuresequenz.

Besonders bevorzugte Ausführungsformen der vorliegenden Erfindung sind die in den Figuren 4 und 5 dargestellten DNA-Sequenzzen, die in Form von PstI-Fragmenten in den Plasmiden pRH 31 und pRH 34 enthalten sind. Das Plasmid pRH 31 ist bei der Deutsche Sammlung für Mikroorganismen (DSM) unter der Hinterlegungs-Nr. DSM 3634 und das Plasmid pRH 34 unter der Hinterlegungs-Nr. DSM 3635 hinterlegt. Die Plasmide pRH 31 und pRH 34 oder davon abgeleitete Fragmente oder synthetische Oligonucleotide sind als Sondenmoleküle zur Identifizierung und Isolierung weiterer DNA-Sequenzen geeignet, die für Proteine mit der biologischen Aktivität der HUSI-TypI-Inhibitoren codieren, also beispielweise für HUSI-I und BSI. Diese Schlußfolgerung ist dem Fachmann aus der hohen Ähnlichkeit der bisher bekannten, jedoch lückenhaften Primärstrukturdaten der Inhibitoren HUSI-I und BSI möglich. Diese lassen nämlich eine hohe Sequenzhomologie auf DNA-Ebene erwarten. Aufgrund der hohen Sequenzhomologien wird auch nicht ausgeschlossen, daß nur ein einziges Gen alle drei Inhibitoren codiert und es sich bei den einzelnen Inhibitoren um gewebespezifische Expressionsprodukte handelt.

Gegenstand der Erfindung sind ferner Vektoren für die Clonierung und Expression der vorstehend genannten DNA-Sequenzen. Erfindungsgemäß bezieht sich der Begriff "Vektoren" beispielsweise auf Plasmide, wie pBR322, pUC18, pUR290, pWH701 und pSP6, oder auf Virusgenome und deren Fragmente oder Derivate, beispielsweise also auf das Genom des lambda-Phagen oder des Phagen M13. In Expressionsvektoren der vorliegenden Erfindung ist die erfindungsgemäße DNA-Sequenz funktionell mit einer Expressionskontroll-Sequenz verbunden. In einer bevorzugten Ausführungsform enthält der Expressionsvektor am 5'-Ende des Gens ein DNA-Fragment mit der Sequenz

```
5' A A T T C G G A G G T G T C G A C T A T G A A G T C C A G C G G 3'
       G C C T C C A C A G C T G A T A C T T C A G G T C G C C
```

Erfindungsgemäß können als Expressionskontroll-Sequenzen (Promotor-Systeme) der E. coli lac-Promotor, der E. coli trp-Promotor, der E. coli Lipoprotein-Promotor, der Promotor der alkalischen Phosphatase, der lambda-$P_L$- oder -$P_R$-Promotor, eine Hefeexpressionskontroll-Sequenz oder eine andere eukaryontische Expressionskontroll-Sequenz verwendet werden.

Die besonders bevorzugten Plasmide der vorliegenden Erfindung sind die Plasmide pRH31 (DSM 3634) und pRH34 (DSM 3635). Weitere besonders bevorzugte Plasmide sind die Plasmide pRH24, pRH21 und pBA17, die mit Hilfe der Plasmide pRH31, pRH34 und pRH1810 (DSM 3905) konstruiert werden können.

Ein weiterer Gegenstand der Erfindung sind Wirtsorganismen, die mit den vorstehend genannten Vektoren transformiert sind. Bevorzugte Wirtsorganismen sind Stämme der Art E. coli, Bacillus subtilis oder andere Bakterien, Saccharomyces cerevisiae, andere mikroskopisch kleine Pilze, tierische oder menschliche Zellen.

Außerdem sind Proteine mit der biologischen Aktivität des CUSI-I-Inhibitors Gegenstand der vorliegenden Erfindung.

In einer besonders bevorzugten Ausführungsform hat das Protein mit der biologischen Aktivität des CUSI-I-Proteins die in Figur 5 dargestellte Aminosäuresequenz.

In einer weiteren besonders bevorzugten Ausführungsform hat das Protein mit der biologischen Aktivität des CUSI-I-Proteins die Aminosäuresequenz

```
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH .
```

In einer weiteren besonders bevorzugten Ausführungsform hat das Protein mit der biologischen Aktivität des CUSI-I-Proteins die Aminosäuresequenz

```
Asp-Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH.
```

Vorzugsweise handelt es sich bei den erfindungsgemäßen Proteinen um praktisch reine Proteine.

Ferner betrifft die Erfindung ein Verfahren zur Herstellung der vorstehend genannten Proteine, bei dem man einen der vorstehend genannten transformierten Wirtsorganismen in einem üblichen Nährmedium züchtet, gegebenenfalls die Expression des Genproduktes induziert, das Expressionsprodukt aus der Kultur, d.h. aus den kultivierten Zellen und/oder aus dem Nährmedium isoliert und gegebenenfalls einer kontrollierten sauren Hydrolyse unterzieht und das gewünschte biologisch aktive Proteinfragment aus dem Hydrolysat durch Gelchromatographie abtrennt.

Je nach Verwendungszweck können die so erhaltenen Proteine weiter gereinigt werden, vorzugsweise durch Chromatographie, wie Affinitätschromatographie oder Hochleistungsflüssigkeitschromatographie (HPLC) oder eine Kombination dieser Methoden.

Die erfindungsgemäß hergestellten Proteine und Proteinfragmente mit HUSI-TypI-Aktivität eignen sich beispielsweise zur Therapie von chronischer Bronchitis, von chronischen Cervixentzündungen, sowie von sonstigen mit exzessiver Schleimbildung verbundenen chronisch-entzündlichen Prozessen und sich daraus ergebenden akuten Notsituationen, weiterhin auch zur Frühtherapie von Schockzuständen und z.B. zur Behandlung von postoperativen Blutungen aufgrund von Hyperfibrinolyse. Dementsprechend sind Arzneimittel mit einem wirksamen Gehalt an einem Protein mit der biologischen Aktivität der HUSI-TypI-Inhibitoren und üblichen Trägerstoffen und/oder Verdünnungsmitteln und/oder Hilfsstoffen ebenfalls Gegenstand der vorliegenden Erfindung.

Zur Therapie kann das Protein mit der biologischen Aktivität des CUSI-I-Inhibitors in Form steriler isotonischer Lösungen durch intramuskuläre, intravenöse oder subkutane Injektionen in das Entzündungsgebiet oder gegebenenfalls durch Infusion verabfolgt werden.

Erfindungsgemäß werden Arzneimittel als Spray oder Inhalationsmittel bevorzugt, die zur Behandlung von Erkrankungen der Atemwege durch direktes Aufbringen der Wirkstoffe auf die betroffenen Teile der Bronchien und Lunge besonders geeignet sind.

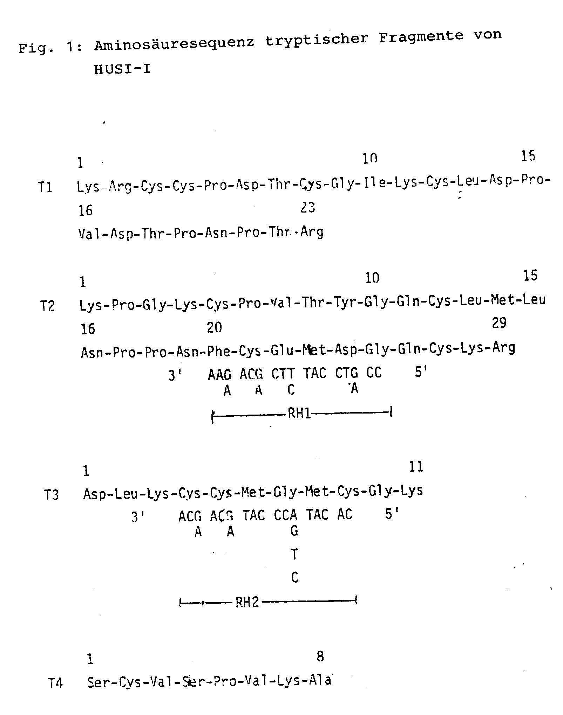

Die Konstruktion eines Wirtsorganismus, der in der Lage ist, ein Fremdprotein zu synthetisieren, umfaßt eine Anzahl experimenteller Schritte. Zunächst wird das Gen, welches die Informationen für die Biosynthese des gewünschten Proteins trägt, identifiziert und isoliert. Es sind verschiedene Verfahren zur Identifizierung und Isolierung von Genen bekannt. Beispielsweise werden zur Isolierung einer DNA-Sequenz, die für ein Protein mit der biologischen Aktivität des CUSI-I-Proteins codiert, basierend auf partiellen Proteinsequenzdaten des HUSI-I-Proteins zunächst zwei Gemische synthetischer Oligonucleotide hergestellt. Diese sind jeweils komplementär zu einer für sechs Aminosäuren codierenden DNA-Sequenz und können als Gensonden verwendet werden (vgl. Figur 1, RHI und RH2).

Ausgehend von den aus dem Stand der Technik bekannten lückenhaften Primärstrukturdaten von CUSI-I, HUSI-I (48) und BSI (41) konnten keine geeigneten Oligonucleotid-Gemische synthetisiert werden. Die bisher bekannten Aminosäuresequenz-Daten wurden nämlich ausgehend von chemisch nicht einheitlich vorliegenden Gemischen von tryptischen Fragmenten, von Bromcyanfragmenten oder von $NH_2$-Termini erstellt (48). Die auf diese Weise erarbeiteten Partialsequenzen weichen außerdem um mehr als 30 % von der erfindungsgemäß ermittelten Aminosäuresequenz des CUSI-I-Proteins ab. Bekanntlich kann jedoch bereits eine einzige falsch bestimmte Aminosäure innerhalb einer Abfolge von Aminosäuren zur Funktionslosigkeit der von dieser Abfolge von Aminosäuren abgeleiteten Oligonucleotid-Sondenmoleküle führen. Aus (48) war z.B. die Aminosäuresequenz Cys-Ser-Met-Gly-Met-Cys im Bereich von RH2 bekannt, die erfindungsgemäß bestimmte Aminosäuresequenz in diesem Bereich ist jedoch Cys-Cys-Met-Gly-Met-Cys (vgl. Figur 4).

Erfindungsgemäß werden mit Hilfe der vorstehend genannten Gemische synthetischer Oligonucleotide cDNA-Genbanken abgesucht. Diese cDNA-Genbanken wurden mit mRNA aus menschlichem Cervix-Gewebe als Ausgangsmaterial hergestellt. Als Ausgangsmaterial zur Herstellung einer erfindungsgemäß geeigneten cDNA-Genbank kann auch mRNA aus dem Gewebe des oberen respiratorischen Traktes der menschlichen Lunge verwendet werden (Autopsiematerial, entnommen 10 Stunden post mortem) (7). Die aus dem Spendergewebe isolierte mRNA wird in üblicher Weise zur Synthese komplementärer DNA (cDNA)-Moleküle verwendet, die schließlich in die PstI-Stelle des Plasmids pBR322 eingebaut werden. Mit den auf diese Weise hergestellten cDNA-Molekülen werden Wirtsorganismen, beispielsweise E. coli K12 DH1 transformiert und in üblicher Weise auf mit Tetracyclin angereicherten Agarplatten ausplattiert. Kolonien transformierter Wirtsbakterien, die ein Plasmid enthalten, das eine cDNA-Sequenz aufweist, die für ein Protein mit der biologischen Aktivität des CUSI-I-Proteins codiert, werden in einem Hybridisierungsexperiment, einer sogenannten Koloniehybridisierung, identifiziert. Für dieses Experiment wird von den auf den Agarplatten wachsenden Bakterienkolonien ein Replika-Nitrocellulosefilter hergestellt, vgl. Thayer (8). Die Replika-Nitrocellulosefilter werden dann mit den beiden vorstehend genannten Oligonucleotid-Gemischen nach Wallace hybridisiert (9). Aus den positiven Kolonien wird das rekombinante, cDNA-enthaltende Plasmid isoliert. Die Größe der cDNA-Insertion des Plasmides wird bestimmt und ein Plasmid mit einer Insertion geeigneter Größe wird durch die Bestimmung der DNA-Sequenz der cDNA-Insertion näher charakterisiert. Auf diese Weise wird das rekombinante Plasmid pRH 31 isoliert, das bei der Deutsche Sammlung für Mikroorganismen unter der Hinterlegungs-Nr. DSM 3634 hinterlegt ist.

Durch ein weiteres Absuchen einer aus mRNA von Cervix-Gewebeproben hergestellten cDNA-Genbank mit einem von der DNA-Sequenz des Plasmids pRH 31 abgeleiteten Oligonucleotid als Sondenmolekül bei der Hybridisierung wird das rekombinante Plasmid pRH 34 isoliert (vgl. Figur 4, RH5). Die Bestimmung der DNA-Sequenz der Insertion des rekombinanten Plasmids pRH 34 zeigt, daß dieses Plasmid den gesamten für das CUSI-I-Protein codierenden Bereich enthält.

Das rekombinante Plasmid pRH 34 ist bei der Deutsche Sammlung für Mikroorganismen unter der Hinterlegungs-Nr. DSM 3635 hinterlegt.

Mit Hilfe der in den Plasmiden pRH 31 und pRH 34 vorliegenden cDNA-Sequenzen werden anschließend Expressionsvektoren konstruiert.

Zunächst wird über das rekombinante Plasmid pRH 1810 als Zwischenprodukt das rekombinante Plasmid pRH 24 hergestellt.

Das rekombinante Expressionsplasmid pRH 24 enthält den für ein Protein mit der biologischen Aktivität des CUSI-I-Proteins codierenden Bereich des Plasmids pRH 34 sowie ein synthetisch hergestelltes DNA-Fragment, das sowohl die Shine-Dalgarno-Sequenz als auch den Translationsstartpunkt enthält. Das ausgehend vom rekombinanten Expressionsvektor pRH 24 hergestellte Expressionsprodukt umfaßt alle in Figur 5 gezeigten Aminosäuren.

Als weiteres rekombinantes Expressionsplasmid wird das Plasmid pRH 21 hergestellt. Dazu wird aus dem Plasmid pRH 31 ein SauIIIA-Fragment ausgeschnitten und in die BamHI-Spaltstelle des Plasmids pUR290 eingesetzt. Das ausgehend von dem so konstruierten rekombinanten Expressionsplasmid pRH 21 hergestellte Expressionsprodukt ist ein Fusionsprotein, dessen N-Terminus aus der Aminosäuresequenz der β-Galaktosidase besteht und dessen 59 C-terminale Aminosäuren den letzten 59 Aminosäuren des CUSI-I-Proteins entsprechen (vgl. Figur 5). Durch saure Hydrolyse der Asparaginsäure-Prolin-Bindung in üblicher Weise (z.B. durch 20- bis 40-stündige Einwirkung von 10- bis 70%iger Essig- oder Ameisensäure vorzugsweise etwa 30%iger Essigsäure oder 70%iger Ameisensäure bei Temperaturen im Bereich von etwa 10 bis 30°C, vorzugsweise bei Raumtemperatur), wird aus diesem Expressionsprodukt ein 58 Aminosäuren langes Polypeptid abgetrennt, das anschließend gelchromatographisch gereinigt wird. Dieses 58 Aminosäuren umfassende Polypeptid entspricht der C-terminalen Domäne des in Figur 5 beschriebenen Proteins mit der biologischen Aktivität des CUSI-I-Proteins.

Als weiteres rekombinantes Expressionsplasmid wird das Plasmid pBA17 konstruiert. Dazu wird aus dem Plasmid pRH1810 ein BamHI/Hinfl-Fragment ausgeschnitten und nach Auffüllung der Enden in das Expressionsplasmid pSP6 ligiert. Das Expressionsplasmid wurde durch eine HindIII-Spaltung und anschließende Behandlung mit "Mungbean" (Mungobohnen)-Nuclease und mit alkalischer Phosphatase für die Ligierung vorbereitet. Das ausgehend von dem erhaltenen rekombinanten Expressionsplasmid pBA17 erhaltene Expressionsprodukt besteht aus 59 Aminosäuren. Seine Sequenz entspricht der der 59 C-terminalen Aminosäuren in Figur 5. Das Expressionsprodukt hat die biologische Aktivität des CUSI-I-Proteins.

Die Expression der vorstehend genannten Proteine durch die entsprechenden transformierten Wirtsorganismen wird anhand einer Immunpräzipitation (10) oder durch eine Westernblot-Analyse (11, 12) nachgewiesen. Die biologische Aktivität der Expressionsprodukte wird durch die Inhibition der Proteinase Chymotrypsin bestimmt.

Die Figuren zeigen:

Figur 1:    Aminosäuresequenz der über HPLC gereinigten Bruchstücke des natürlichen CUSI-I-Proteins, die nach enzymatischer Spaltung des Proteins mit Trypsin erhalten wurden. Die Sequenz-Bereiche, von denen die beiden Gemische synthetischer Oligonucleotide abgeleitet wurden, sind mit RH1 und RH2 bezeichnet und unterstrichen.

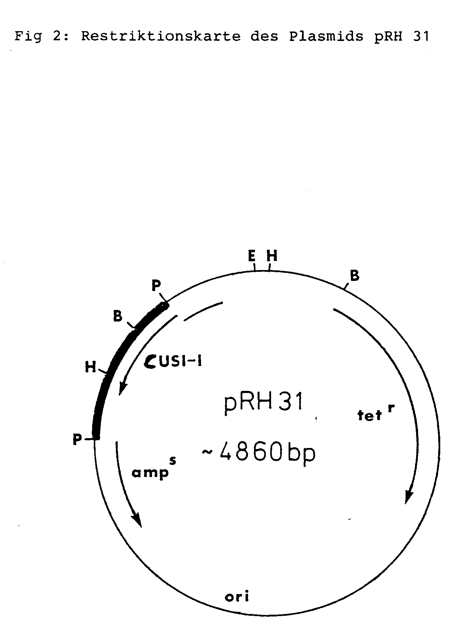

Figur 2:    Restriktionskarte des Plasmids pRH 31 Das rekombinante Plasmid pRH 31 ist mit den Restriktionsspaltstellen für P = PstI, E = EcoRI, B = BamHI, H = HindIII dargestellt. Der schwarze Balken bezeichnet die Lage der cDNA-Insertion, die Pfeile innerhalb des Kreises kennzeichnen die Lage des Tetracyclin-Resistenzgens (tet$^r$) und des unterbrochenen Ampicillin-Resistenzgens(amp$^s$).

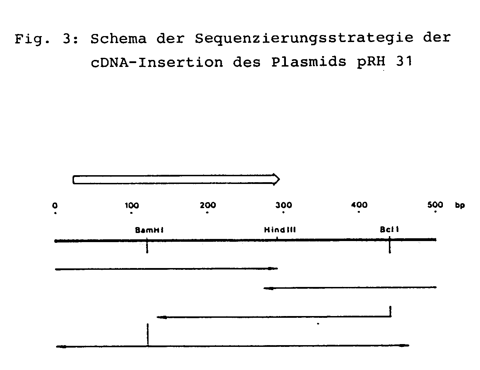

Figur 3:    Schema der Sequenzierungsstrategie der cDNA-Insertion des Plasmids pRH 31 Der schwarze Balken stellt das 500bp-PstI-Fragment des Plasmids pRH 31 dar, die schwarzen Pfeile entsprechen jeweils einer Sequenzierungsreaktion. Durch den weißen, nicht ausgefüllten Pfeil ist der für CUSI-I codierende Bereich auf der cDNA-Insertion gekennzeichnet.

Figur 4:    Nucleotidsequenz des CUSI-I-cDNA-Fragmentes des Plasmids pRH 31. Die DNA-Sequenz ist doppelsträngig dargestellt. Das offene Leseraster der CUSI-I-Sequenz beginnt direkt nach dem 5'-terminalen-(G:C)- homopolymeren Ende der cDNA-Insertion. Das Leseraster codiert 90 Aminosäuren, die im Dreibuchstabencode angegeben sind. Ferner sind 178 Basenpaare dargestellt, die sich an das Stopcodon TGA anschließen.

Figur 5:    Nucleotidsequenz des CUSI-I-cDNA-Fragmentes des Plasmids pRH 34. Die DNA-Sequenz ist doppelsträngig dargestellt. Die von der DNA-Sequenz abgeleiteten Aminosäuren sind in Dreibuchstabencode angegeben. Die Nucleotide von Position 59 bis 133 codieren das Signalpeptid des CUSI-I-Proteins.

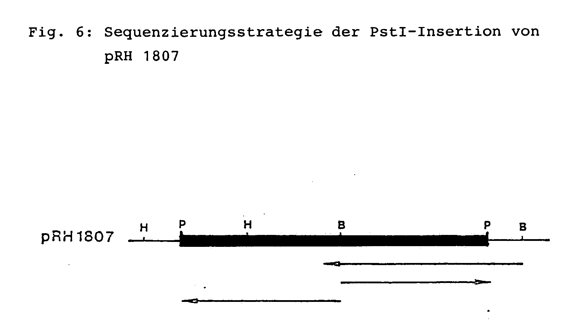

Figur 6:    Sequenzierungsstrategie der PstI-Insertion von pRH 1807 (enthaltend das CUSI-I-cDNA-Fragment des Plasmids pRH 34) Jeder Pfeil gibt ein Sequenzierungsexperiment an. H = HindIII, P = PstI, B = BamHI.

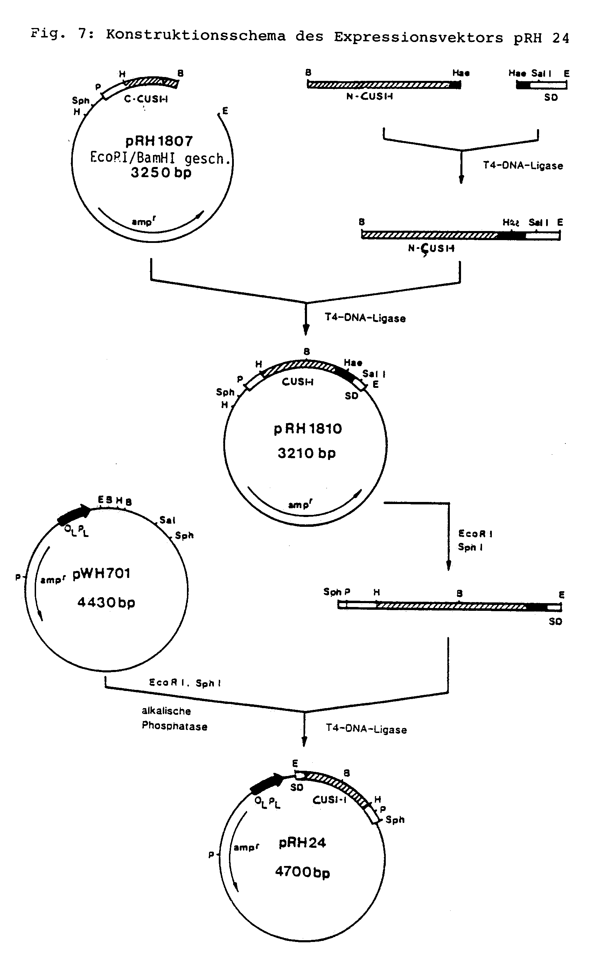

Figur 7:    Konstruktionsschema des Expressionsvektors pRH 24, der das CUSI-I-Gen am 3'-Ende des regulierbaren Promotors lambda-P$_L$ trägt

amp$^r$ :    Ampicillin-Resistenzgen

N-CUSI-I :    für den CUSI-I-N-Terminus codierendes DNA-Fragment

C-CUSI-I :    für den CUSI-I-C-Terminus codierendes DNA-Fragment

O$_L$P$_L$ :    Linke Operator- und Promotor-Region des Bakteriophagen lambda

SD :    Shine-Dalgarno-Sequenz bzw. Ribosomenbindungsstelle Abkürzungen der Restriktionsendonucleasen: B = BamHI, E = EcoRI, H = HindIII, Hae = HaeIII, P = PstI, Sph = SphI

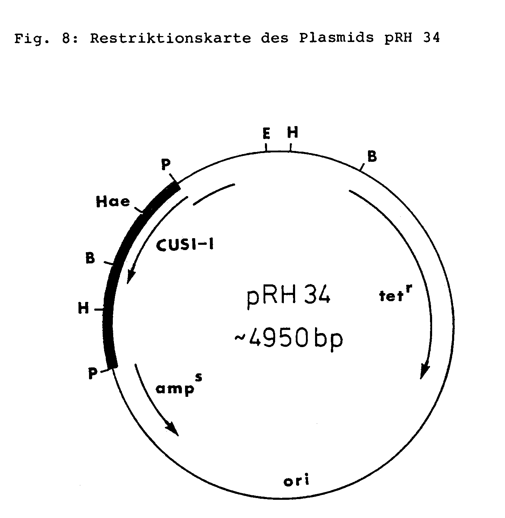

Figur 8:    Restriktionskarte des Plasmids pRH 34 Das rekombinante Plasmid pRH 34 ist mit den Restriktionsspaltstellen für P = PstI, E = EcoRI, B = BamHI, H = HindIII, Hae = HaeIII dargestellt. Der schwarze Balken zeigt die Lage der cDNA-Insertion, die Pfeile innerhalb des Kreises kennzeichnen die Lage des Tetracyclin-Resistenzgens (tet$^r$) und des zerstörten Ampicillin-Resistenzgens (amp$^s$)

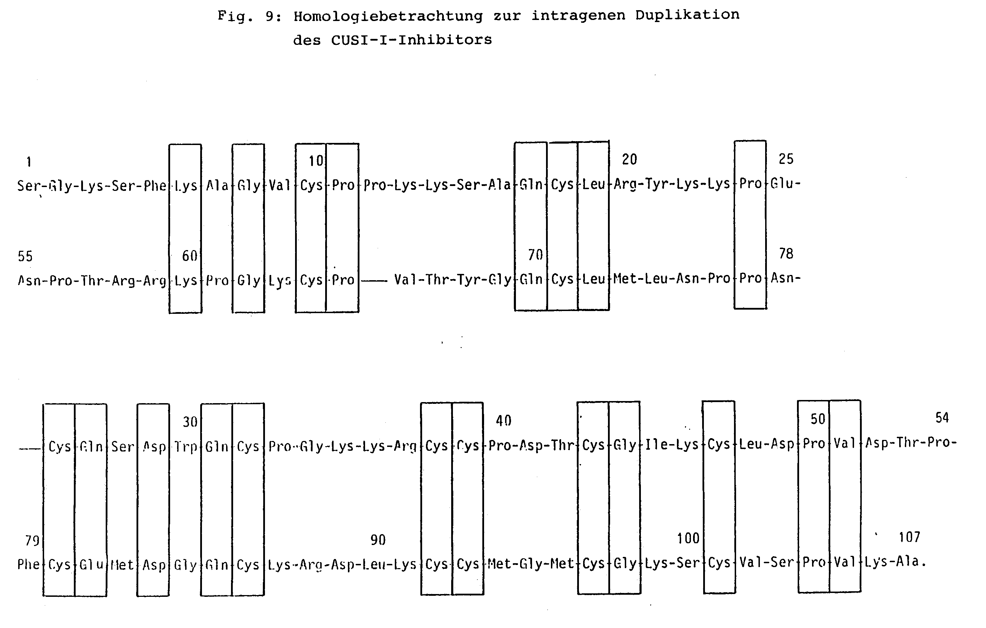

Figur 9:    Eine Analyse der Aminosäure-Sequenz wirft einen interessanten Aspekt auf: Unter Vernachlässigung von zwei Verschiebungen der Sequenz, lassen sich sämtliche im Molekül enthaltene Cysteinreste übereinander projizieren, wenn man das Protein in zwei Hälften aufteilt und übereinander schreibt (Aminosäure 1-54 bzw. 55-107). Ferner fällt

7

auf, daß benachbarte Aminosäuren bezogen auf die Cysteinreste häufig konserviert sind. Diese Beobachtung impliziert zwei Modellvorstellungen:

1. CUSI könnte aus zwei fast identisch gefalteten hemmaktiven Domänen bestehen, eine für Trypsin und eine für Leukozytenelastase bzw. Chymotrypsin.

2. Das Protein hat sich eventuell auf der Ebene des genetischen Codes aus einer vorhandenen "Domäne" gebildet, wobei sich beide Domänen dann unabhängig voneinander weiterentwickelt haben.

Figur 10: Clonierungsstrategie zur Expression von CUSI-I-Partialsequenzen als β-Galaktosidase-Fusionsprotein. Der schwarze Balken kennzeichnet CUSI-I-Partialsequenzen, der nicht ausgefüllte Balken das β-Galaktosidasegen (lacZ), und die Pfeile innerhalb des Kreises zeigen die Lage des Tetracyclin-Resistenzgens (tet$^r$) bzw. des Ampicillin-Resistenzgens (amp$^r$) an. P = PstI, S = Sau3A, E = EcoRI, H = HindIII, B = BamHI, p = lac-Promotor, o = lac-Operator.

Figur 11: Konstruktionsschema des Expressionsvektors pBA17 zur Expression der C-terminalen CUSI-I-Domäne ( = CUSI-I 2nd domain)

Amp$^r$ = Ampicillin-Resistenzgen

P$_{tac}$ = tac-Promotor

Tet$^s$ = inaktives Tetracyclinresistenz-Restgen

rrnBT$_1$T$_2$ = Transcriptions-Terminator-Signal von ribosomalen RNA-Genen

Die nachstehend benutzten Abkürzungen haben folgende Bedeutungen:

A$_{578}$    Absorption bei 578 nm

DTT    Dithiothreitol

Bis    N,N,N',N'-Methylenbisacrylamid

bp    Basenpaare

D    Dalton

DE (DEAE)    Diäthylaminoäthyl

ds cDNA    doppelsträngige cDNA

dNTP    Desoxynucleosid-5'-triphosphat

EDTA    Äthylendiamintetraessigsäure

IgG    Immunglobuline

IPTG    Isopropyl-β-D-thiogalactopyranosid

$^{32}$P    Phosphorisotop der relativen Masse 32

Upm    Umdrehung pro Minute

$^{35}$S    Schwefelisotop der relativen Masse 35

SDS    Natriumdodecylsulfat

ss cDNA    einzelsträngige cDNA

TPEG    p-Aminophenyl-I-thio-β-D-galactopyranosid

Tris    Trishydroxymethylaminomethan

U    Einheit der Enzymaktivität

Sarkosyl    N-Laurylsarkosin

α-[$^{32}$P]-dCTP    Desoxycytidyl-5'-triphosphat mit dem Isotop $^{32}$P in der α-Phosphatgruppe

LB-Medium    10 g/l Casein enzymatisch gespalten (Sigma), 5 g/l Hefeextrakt (Sigma) 8 g/l NaCl auf pH 7,5 eingestellt.

In den Ausführungsbeispielen wird nach den nachstehend beschriebenen Methoden unter Verwendung der genannten Materialien verfahren. Die Materialien und Methoden sind ferner in T. Maniatis et al. (28) beschrieben.

## 1. Enzyme

Restriktionsendonucleasen (Bethesda Research Laboratory (BRL)) und T4-DNA-Ligase (Boehringer Mannheim) sowie alkalische Phosphatase aus Kälberdarm (Boehringer Mannheim), T4-Polynucleotidkinase (Boehringer Mannheim) und "Mungbean" (Mungobohnen)-Nuclease (Pharmacia) sind handelsüblich und werden nach den Angaren der Hersteller benutzt. Terminale Desoxynucleotidyl-Transferase (BRL) wird, wie in Abschnitt 7 beschrieben, eingesetzt. E. coli DNA-Polymerase (BRL), Ribonuclease H (BRL) sowie AMV Reverse Transcriptase (Life Science) werden entsprechend (13) eingesetzt. E. coli DNA-Polymerase I (Klenow Fragment (Boehringer Mannheim)) wird wie in (14) beschrieben eingesetzt.

2. Mikroorganismen

Als Mikroorganismen für die Expression eines Proteins mit der biologischen Aktivität des CUSI-I kommen sowohl gram-negative als auch gram-positive Stämme wie Stämme von E. coli oder von Bacillus subtilis in Betracht.

Ebenso können mit geeigneten, das Strukturgen für ein Protein mit der biologischen Aktivität des CUSI-I enthaltenden Vektoren die gebräuchlichen Expressionssysteme für Eukaryonten benutzt werden, beispielsweise Saccharomyces cerivisiae oder Säugerzellen.

Für die erfindungsgemäße direkte Expression des vollständigen natürlichen CUSI-I-Proteins wird der E. coli Stamm K 12 MC 1061 $\lambda$15 (hinterlegt bei der DSM unter der Hinterlegungsnummer DSM 3631) benutzt. Sein Genotyp läßt sich folgendermaßen definieren: araD139, $\Delta$(ara, leu)7697, $\Delta$lacX74, galU$^-$, galk$^-$, hsr$^-$, hsm$^+$, strA.

Für die erfindungsgemäße Expression des Fusionsproteins zwischen der $\beta$-Galaktosidase und der C-terminalen Domäne des CUSI-I-Proteins wird der E.coli Stamm K12 JM 101 (hinterlegt bei der American Type Culture Collection (ATCC) unter der Hinterlegungs-Nr. ATCC 33876) benutzt. Sein Genotyp läßt sich wie folgt definieren: $\Delta$(lac pro), thi, strA, supE, endA, sbcB, hsdR$^-$, F'tra D36, pro AB, lacI$^q$, Z $\Delta$ M15.

Für die Isolierung rekombinanter Plasmide, die nach der Insertion synthetischer cDNA erhalten wurden (vgl. auch Beispiel 1c)], wird E. coli K12 DH 1 (hinterlegt bei der ATCC unter der Hinterlegungs-Nr. 33849) (17) benutzt. Sein Genotyp läßt sich wie folgt definieren: F$^-$, recA1, endA1, gyrA96, thi-1, hsdR17 ($r_k^-$ , $m_k^+$ ), supE44, relA1.

Zur Isolierung von Plasmiden und neu konstruierten rekombinanten Plasmiden, die den Promotor $p_L$ des lambda-Phagen enthalten, wird zunächst in E. coli K12 Wildtyp W6 Bakterien transformiert. In diesen Wirtsbakterien ist der lambda-$p_L$-Promotor durch einen thermostabilen lambda-Repressor konstant blockiert. E. coli K12 Wildtyp W6 ist hinterlegt bei der DSM unter der Hinterlegungsnummer DSM 3632 .

3. Vektoren

Für die Transformation von E. coli-Bakterien werden folgende bekannte und teilweise im Handel erhältliche Plasmide benutzt.

pBR 322        (ATCC [31344], (23), Pharmacia, Freiburg),
pUC 18         (hinterlegt bei der Deutsche Sammlung für Mikroorganismen unter der Hinterlegungs-Nr. DSM 3424, (24), Pharmacia, Freiburg),
puR 290        (DSM 3417, (25))
pWH 701        (DSM 3633, (26))
pRK 248        clts in E.coli K12 JMB9 (ATCC 33766, (27), (49)),
pSP6           (DSM 3904) und pRH 1810 (DSM 3905).

Dem Fachmann sind weitere geeignete Vektoren bekannt, die in Verbindung mit ihm ebenfalls bekannten Wirtsorganismen erfindungsgemäß verwendet werden können.

4. Gelelektrophoresen

Zur Trennung von DNA-Fragmenten werden je nach Größe der DNA Agarose bzw. Polyacrylamidgele verwendet. DNA-Fragmente > 800 bp werden auf 1 - 1,2prozentigen Agarosegelen in TAE-Puffer (40 mM Tris-Acetat, pH 8, 3, 2 mM EDTA), DNA-Fragmente < 800 bp in 5prozentigen Polyacrylamidgelen (Acrylamid/Bis-Acrylamid (19:1) mit TBE Puffer (60 mM Tris-Base, 60 mM Borsäure, 1 mM EDTA, pH 8,3) getrennt. Denaturierende Agarose-Gelelektrophoresen zur Trennung von cDNA erfolgen z.B. in 1,2prozentigen alkalischen Agarosegelen nach McDonnell et al. (19).

Zur gelelektrophoretischen Trennung von mRNA werden 1,4prozentige Agarosegele mit 15 mM Methylquecksilberhydroxid verwendet. Die Elektrophorese und Denaturierung der Proben kann nach Bailey et al. (20) durchgeführt werden. Die Trennung von Proteinen in SDS-Polyacrylamidgelen erfolgt nach Laemmli (21).

5. Gelelutionsmethoden

Die präparative Trennung von DNA-Fragmenten < 1.000 bp wird in 5 % Polyacrylamidgelen durchgeführt. Die Elution der Fragmente erfolgt nach Maxam und Gilbert (22). Auf diese Weise isolierte Fragmente wurden zur Umklonierung, bzw. zur Sequenzanalyse eingesetzt.

6. Isolierung von RNA

Die Isolierung von Gesamt-RNA aus menschlichem Gewebe erfolgt nach Maniatis et al. (28). An die Extraktion der Gesamt-RNA aus dem Gewebe schließt sich eine CsCl-Gradientenzentrifugation an, bei der die DNA abgetrennt wird (29). Dazu werden in einem 16 ml Beckman-SW27-Zentrifugenröhrchen 3 ml 5,7 M CsCl,100 mM EDTA vorgelegt, mit 4 ml RNA-Lösung (3 - 4 mg Nucleinsäure in 1 % N-Laurylsarkosin, 5 mM Tris-HCl pH 7,5, 1 mM EDTA, 4 g/4 ml CsCl) überschichtet, mit Puffer (1 % N-Laurylsarkosin, 5 mM Tris-HCl, pH 7,5, 1 mM EDTA) aufgefüllt und im Beckman SW 27-Rotor für 17 h bei 15°C mit 17.000 U/min zentrifugiert. Das RNA-Sediment wird in TE-Puffer resuspendiert (10 mM Tris-HCl,pH 8,0,1 mM EDTA) , mit Äthanol gefällt und anschließend zur Anreicherung von poly (A$^+$)-mRNA an Oligo(dT)-Cellulose (Typ 9, Pharmacia, Freiburg) nach Aviv und Leder (30) chromatographiert. Die mRNA wird mit Äthanol aus dem Eluat gefällt und bei -70°C in 70 % Äthanol aufbewahrt. Die Intaktheit des mRNA-Isolates kann man durch in vitro-Translation im Kaninchen-Retikulozytenlysat (vgl. Abschnitt 16) bzw.denaturierende Gelelektrophorese überprüfen. Auf diese Weise isolierte mRNA wird anschließend zur cDNA-Synthese sowie zur Northern-Blot-Analyse eingesetzt.

7. Synthese von cDNA

Die Synthese von komplementärer einzelsträngiger und doppelsträngiger DNA wird nach Gubler et al. (13) durchgeführt. Die Ausbeute der Synthese wird durch den Einbau von α-[$^{32}$P]-dCTP verfolgt, während die Länge der entstandenen cDNA-Moleküle mit einem 1,2prozentigen alkalischen Agarosegel und radioaktiv markierten Standard-DNA-Molekülen ermittelt wird. Zur Addition von Oligo-(dc)-Homopolymeren an die 3'-Enden der cDNA verfährt man z.B. in 40 $\mu$l Gesamtvolumen unter folgenden Bedingungen: 1oo mM K-Kakodylat, pH 7,2, 10 mM CoCl$_2$, 1 mM DTT, 500 $\mu$M dCTP, 1 - 2 mg/ml cDNA, 600 U/ml terminale Desoxynucleotidyl-Transferase. Das Reaktionsgemisch wird 50 min bei 20°C inkubiert und die Reaktion durch Zugabe von EDTA (Endkonzentration 20 mM) abgestoppt. Nach Äthanolfällung aus einer 0,3 M Na-Acetatlösung wird die präzipitierte cDNA in TE-Puffer (10 mM Tris-HCl, pH 8,0, 1 mM EDTA) suspendiert und mit PstI-geschnittenem, 3'-Oligo(dG)-verlängertem Plasmid pBR322 hybridisiert (100 mM NaCl, 10 mM Tris-HCl pH 7,8, 0,1 mM EDTA, 0,1 - 0,3 ng/$\mu$l cDNA, 1,2 ng/$\mu$l Plasmid-DNA). Zur Hybridisierung wird dieses Gemisch nacheinander 5 min bei 65°C, 45 min bei 56°C, 45 min bei 43°C und 15 min bei Raumtemperatur inkubiert und anschließend direkt zur Transformation (vgl. Abschnitt 9) eingesetzt.

8. Northern-Blot-Analyse

Die denaturierende Gelelektrophorese der RNA und der Transfer der RNA auf Nitrocellulosefilter wird nach Thomas (31) durchgeführt. Prähybridisierung und Hybridisierung mit 5'-markierten Oligonucleotiden können nach der in (9) beschriebenen Methode erfolgen ( vgl. Abschnitt 11). Unspezifisch gebundene Oligonucleotide kann man nach der Hybridisierung durch Waschen der Nitrocellulosefilter entfernen, beispielsweise dadurch, daß man die Filter 15 Minuten bei Raumtemperatur und 3 Minuten bei 2°C unterhalb der Schmelztemperatur in SSC-Puffer (900 mM NaCl, 90 mM Natriumcitrat, pH 7,0) wäscht. Die Schmelztemperatur wird dabei nach Suggs et al. (32) berechnet.

9. Transformation von E. coli und Isolierung der Plasmide

Zur Transformation mit rekombinanten Plasmiden, die neu synthetisierte cDNA enthalten (vgl. Beispiel 1, c)), werden kompetente Zellen von E.coli K12 DH1 nach Hanahan (17) hergestellt. Die Transformation von Zellen der E. coli K12 Stämme JM101, W6 und Mc1061 wird nach Mandel und Higa (33) durchgeführt. Die Präparation von Plasmid-DNA aus 1 Liter Kulturen erfolgt nach der in (34) beschriebenen Methode. Plasmid-Schnellanalysen werden nach Holmes et al. (35) durchgeführt.

10. Oligonucleotidsynthese

Oligonucleotide werden nach der Phosphoamidit-Methode synthetisiert (36). Die Reinigung der Oligonucleotide erfolgt beispielsweise durch Reversed-Phase-Chromatographie an Shandon-Hypersil ODS $^R$-(Partikelgröße 5 $\mu$m, Säulengröße 4,6 x 250 mm). Nach Abspaltung der Tritylgruppe mit 80prozentiger Essigsäure werden die Produkte an dem genannten Säulenmaterial nochmals chromatographiert und nach der Markierung am 5'-Ende (vgl. Abschnitt 11) in 20prozentigen Polyacrylamidgelen analysiert.
Nach diesem Verfahren werden zwei Oligonucleotid-Gemische, nämlich RH1 und RH2 synthetisiert. Diese Oligonucleotid-Gemische werden jeweils als Sondenmoleküle verwendet. Die Sequenzen der Oligonucleotid-Moleküle in den beiden Gemischen werden aus geeigneten Aminosäuresequenzen von tryptischen Fragmenten des HUSI-I-Proteins abgeleitet (vgl. Figur 1). Die Aminosäuresequenzen dieser Fragmente wurden nach W. Machleidt (5o) bestimmt. Erst die Korrektur bekannter HUSI-I-Sequenzen durch die Analyse von mehreren tryptischen Fragmenten und von mehreren Bromcyanfragmenten ermöglichte die Ableitung dar erfindungsgemäßen Oligonucleotidgemische.

Es handelt sich also bei den Oligonucleotid-Gemischen um sogenannte "mixed probes", d.h. um Gemische von Oligonucleotiden, die sich in definierten Positionen aufgrund der Degeneration des genetischen Codes unterscheiden (9). Durch die HPLC-Hochreinigung der Oligonucleotide sowie durch ihre quantitative $^{32}$P-Phosphorylierung konnten die Nachteile üblicher "mixed probes" kompensiert werden.

Das Oligonucleotid-Gemisch RH1 entspricht einer Aminosäuresequenz aus dem tryptischen Fragment T2 des CUSI-I-Proteins (vgl. Figur 1). Insgesamt enthält das Oligonucleotid-Gemisch somit 16 verschiedene Oligonucleotide, die jeweils 17 Basen lang sind. Die Sequenzen sind die folgenden:

```
3'    A A G A C G C T T T A C C T G C C   5'
              A       A       C               A
```

Das Oligonucleotid-Gemisch RH2 entspricht einer Aminosäuresequenz aus dem tryptischen Fragment T3 des HUSI-I-Proteins (vgl. Figur 1). Es werden somit 32 verschiedene Oligonucleotide synthetisiert, die jeweils 17 Basen lang sind. Sie haben die folgenden Sequenzen

```
3'    A C A A C A T A C C A T A C A C   5'
          G       G               G
                                  C
                                  T
```

## 11. Radioaktive Markierung von DNA

Die Phosphorylierung chemisch synthetisierter Oligonucleotide und doppelsträngiger dephosphorylierter DNA-Fragmente erfolgt mit Hilfe des Enzyms T4-Polynucleotid-Kinase in 20 bis 50 $\mu$l Reaktionsvolumen (50 mM Tris-HCl pH 9,5, 20 mM MgCl$_2$, 1 mM EDTA, 10 - 20 pmol 5'-OH-Enden Substrat, 8 $\mu$l $\gamma$-[$^{32}$P]-ATP ($\sim$ 8000 Ci/mmol), 0,2 U/$\mu$l T4-Polynucleotid-Kinase, die anschließende Trennung von nicht umgesetztem $\gamma$-[$^{32}$P]-ATP durch präparative Gelelektrophorese, anschließender Gelelution und Ionenaustauschchromatographie an DE-52® Diaminodiäthyl-Cellulose) (Whatman). 5'-überstehende Enden von DNA-Restriktionsfragmenten lassen sich mit dem Klenow-Fragment der E. coli DNA-Polymerase I in Gegenwart der jeweils komplementären $\alpha$-[$^{32}$P]-Desoxyribonucleosidtriphosphate nach Volkert et al. (14) auffüllen. Nicht eingebaute $\alpha$-[$^{32}$P]-dNTPs werden durch Gelpermeationschromatographie an Sephadex® G-50 abgetrennt und eluierte Fraktionen unter vermindertem Druck eingeengt.

## 12. DNA-Sequenzanalysen

Die Sequenzierung von DNA-Molekülen wird nach Maxam und Gilbert (22) vorgenommen.

## 13. Reinigung des $\beta$-Galactosidase-Fusionsproteins

Das CUSI-I-$\beta$-Galactosidase-Fusionsprotein wird affinitätschromatographisch nach Ullmann (37) gereinigt.

## 14. Transfer von Proteinen auf Nitrocellulosefilter

In SDS-Polyacrylamidgelen getrennte Proteine werden nach Towbin eb al. (12) auf Nitrocellulosefilter transferiert.

15. CUSI-I-Inhibitionstests

Die Aktivität von Proteinen mit der biologischen Aktivität des CUSI-I wird durch Messung der Inhibition von Trypsin (38) und Chymotrypsin (39) nachgewiesen.

16. Zellfreie Translation von mRNA

Die zellfreie Translation von mRNA im Retikulozytenlysat wird nach Pelham et al. (40) unter Verwendung von $^{35}$S-Methionin mit einer spezifischen Aktivität von 1200 Ci/mmol als radioaktiv markierter Aminosäure durchgeführt.

Die Beispiele erläutern die Erfindung.

**Beispiel 1**

Clonierung und Identifizierung eines partiellen CUSI-I-Protein-spezifischen cDNA-Clons

a) Isolierung und Charakterisierung von mRNA

Gesamt-RNA bzw. mRNA wird wie oben in Abschnitt 6 beschrieben aus menschlichem Cervix gewebe (Biopsiematerial) isoliert. Aus etwa 14 bis 17 g Cervixgewebe werden etwa 1,6 bis 2,2 mg Gesamt-RNA gewonnen. Nach Anreicherung von poly (A$^+$)-mRNA durch Affinitätschromatographie an Oligo(dT)-Cellulose (Typ 9, Pharmacia) werden 20 bis 25 $\mu$g mRNA/mg Gesamt-RNA erhalten. Dies entspricht einer Ausbeute von etwa 2,0 bis 2,5 % mRNA. Eine gelelektrophoretische Analyse im denaturierenden Agarosegel zeigt, daß die RNA während der Isolierung nicht abgebaut wird. Entsprechende Resultate liefert auch eine in vitro-Translation der Gesamt- und poly(A$^+$)-mRNA im Kaninchen-Retikulozytenlysat.

b) Northern-Blot-Analyse der isolierten mRNA mit einem synthetischen Oligonucleotid zum Nachweis von CUSI-I-Proteinspezifischen Sequenzen.

Zur Identifizierung spezifischer mRNA-Sequenzen im Cervix-mRNA-Isolat wird eine Northern-Blot-Analyse durchgeführt. Als Hybridisierungssonde dient dabei das Oligonucleotid-Gemisch RH1, das komplementär zu einer mRNA ist, die für die Aminosäuren Phe-Cys-Glu-Met-Asp-Gly codiert (vgl. Figur 1). Durch Belichtung eines Röntgenfilms ("Kodak X-o-mat-AR") mit einem bei der Hybridisierung erhaltenen Nitrocellulosefilter wird ein spezifisches Signal nachgewiesen.

Mit dem Oligonucleotidgemisch RH2 wurde kein positives Signal gefunden obwohl sich eine der Oligonucleotidsequenzen des Gemisches später (nach der Sequenzierung)als richtig herausstellte.

Aus einem Vergleich der Molekülgröße der hybridisierenden mRNA-Spezies mit DNA-Standardmolekülen in einem denaturierenden Gel ergibt sich, daß die Länge der hybridisierenden mRNA etwa 650 bis 800 Basen beträgt.

c) Herstellung einer cDNA-Bank mit dem Plasmid pBR322

Für die Synthese von cDNA werden 4 bis 6 $\mu$g poly(A$^+$)-mRNA als Ausgangsmaterial eingesetzt. Die Einzelstrang-cDNA-Ausbeute beträgt etwa 280 ng (etwa 7 %). Die synthetisierten einzelsträngigen cDNA-Moleküle haben eine Größe von etwa 400 bis 2500 Nucleotiden. Aus 280 ng ss cDNA werden bei der Synthese des zweiten Stranges ca. 270 ng doppelsträngige cDNA erhalten. Somit beträgt die Ausbeute bei der Doppelstrangsynthese etwa 50 %. An die 3'-Enden der cDNA-Moleküle werden homopolymere (dC)-Bereiche angehängt. Die so erhaltenen cDNA-Moleküle werden an am 3'-Ende mit homopolymeren (dG)-Bereichen verlängerte und PstI-gespaltene Moleküle des Plasmids pBR322 in einer Hybridisierungsreaktion angelagert. Die Anlagerungsprodukte werden zur Transformation von E. coli K12 DH1 verwendet. Anschließend erfolgt eine Selektion der Transformanten auf Tetracyclin-Resistenz und Ampicillin-Sensitivität. Pro ng eingesetzter cDNA werden 120 Transformanten (12 000 Transformanten/100 ng ds cDNA) erhalten. Der Anteil der Tetracyclin-resistenten und Ampicillin-sensitiven Transformanten bzw. Kolonien beträgt etwa 80 %. Diese Transformanten werden in Mikrotiterplatten (98 Bohrungen/Platte) als Dauerkulturen ausplattiert (Medium: LB-Medium, 10 g/l Casein enzymatisch gespalten (Sigma), 8 g/l NaCl, pH 7,5, 5 g/l Hefeextrakt (Sigma), 20 $\mu$g/ml Tetracyclin, 20 % Glycerin) und bei -20ºC aufbewahrt.

d) Identifizierung eines rekombinanten Plasmids mit einer CUSI-I-Protein-spezifischen cDNA

Zur Analyse von 6000 der wie vorstehend beschrieben erhaltenen Transformanten wird eine Koloniehybridisierung mit dem Oligonucleotid-Gemisch RH 1 durchgeführt. Die Aktivität des Oligonucleotid-Gemischs beträgt etwa 1 x 10⁶ cpm im Hybridisierungsvolumen bei einer spezifischen Aktivität von 0,8 µCi/pmol. Röntgenfilme werden mit den bei der Hybridisierung erhaltenen Filtern jeweils 12 Stunden bei -70°C unter Verwendung von zwei Verstärkerfolien belichtet. Es wird ein positives Signal identifiziert.
Ausgehend von der Dauerkultur der entsprechenden Transformante wird aus einer 0,5 Liter-Kultur (LB-Medium, 10 g/l Casein enzymatisch gespalten (Sigma) 10 g/l NaCl, 5 g/l Hefeextrakt (Sigma), pH 7,5 , 20 µg/ml Tetracyclin) das als pRH 31 bezeichnete rekombinante Plasmid präpariert. Unter Verwendung der Restriktionsendonucleasen Pst I, EcoR I, BamH I sowie Hind III wird eine Restriktionskarte des rekombinanten Plasmids pRH 31 erstellt (vgl. Figur 2). Die cDNA-Insertion beträgt 500 bp (vgl. Figur 2).
Das Plasmid pHR 31 ist bei der DSM unter der Hinterlegungsnummer DSM 3634 hinterlegt.

e) Sequenzanalyse der cDNA-Insertion von pRH 31

Zur Sequenzierung nach Maxam und Gilbert (22) wird das PstI-Fragment (500 bp) aus dem Plasmid pRH 31 in das Plasmid pUC18 umcloniert. Zu diesem Zweck werden 10 µg DNA von pRH 31 mit 30 U der Restriktionsendonuclease PstI gespalten, präparativ an einem Agarosegel getrennt und das erhaltene 500 bp-Fragment wird aus dem Gel eluiert. Außerdem werden 10 µg DNA des Plasmids pUC18 mit der Restriktionsendonuclease PstI gespalten, dephosphoryliert, mit Phenol und Diäthyläther extrahiert und schließlich mit Äthanol aus einer 0,3 molaren Natriumacetat-Lösung ausgefällt. In die anschließende T4-DNA Ligase-Reaktion werden jeweils 0,2 pmol Plasmid-DNA und 0,4 pmol des PstI-Fragments eingesetzt. Die so erhaltenen rekombinanten DNA-Moleküle werden zur Transformation von E. coli K12 JM 101 verwendet. Es wird auf mit Ampicillin angereicherten LB-Platten (10 g/l Casein, 8 g/l NaCl, 5 g Hefeextrakt, 100 µg/ml Ampicillin) selektiert. In Ampicillin-resistenten Transformanten enthaltene rekombinante Plasmide werden durch Plasmid-Schnellanalysen untersucht. Es werden die rekombinanten Plasmide pRH 181 und pRH 182 erhalten, die das PstI-Fragment von pRH 31 in entgegengesetzter Orientierung enthalten. Aus der Konstruktion dieser Plasmide, die lediglich als Hilfskonstruktionen zur einfacheren DNA-Sequenzierung dienen, ergibt sich die in Figur 3 dargestellte Sequenzierungsstrategie.
Die bei der Sequenzierung (22) ermittelte Nucleotidsequenz des 500 bp langen PstI-Fragment aus dem Plasmid pRH 31 ist in Figur 4 dargestellt. Die Sequenz beginnt am 5'-Ende mit 20 (dG)-Resten. Es schließt sich ein offenes Leseraster an, das sich über 273 bp erstreckt (vgl. Figur 4, Positionen 25 bis 297). Dieses offene Leseraster codiert für 90 Aminosäuren und wird von einem Stopcodon TGA abgeschlossen (vgl. Figur 4). Aus der Nucleotidsequenz ist zu entnehmen, daß die Oligonucleotide des Oligonucleotid-Gemisches RH1 komplementär zu den Positionen 208 bis 224 der Nucleotidsequenz sind und die Oligonucleotide des Oligonucleotid-Gemisches RH2 komplementär zu den Positionen 247 bis 263 der Nucleotidsequenz sind.
Auf das Stopcodon TGA folgen weitere 178 bp. Die Analyse der Nucleotidsequenz zeigt, daß der den N-terminalen Abschnitt des CUSI-I-Protein codierende Bereich nicht in der Nucleotidsequenz enthalten ist.

**Beispiel 2**

Isolierung eines rekombinanten Plasmids mit einem cDNA-Fragment, das für das gesamte CUSI-I-Protein codiert

a) Synthese des Oligonucleotids RH5

Zur Isolierung eines cDNA-Fragments, das den gesamten für das CUSI-I-Protein codierenden Bereich enthält, wird das Oligonucleotid RH5 nach der Phosphoamidit-Methode (36) synthetisiert. Das Oligonucleotid RH5 hat eine Länge von 20 Basen. Es ist komplementär zu den Positionen 31 bis 50 des in Figur 4 abgebildeten codierenden DNA-Stranges und hat die folgende Sequenz:

5'                                                3'
C A C T C A G G T T T C T T G T A T C T

Das Oligonucleotid RH5 wird radioaktiv markiert und als Sondenmolekül eingesetzt, um in einer neuen cDNA-Bank Transformanten zu identifizieren, die rekombinante Plasmide enthalten, deren cDNA-Fragmente am 5'-Ende mindestens den 5'-terminalen Bereich des Plasmids pRH 31 enthalten.

b) Herstellung einer neuen cDNA-Genbank, die ein rekombinantes Plasmid mit dem gesamten codierenden Bereich des CUSI-I-Proteins enthält.

Zur Anlage einer neuen cDNA-Bank in E. coli mit dem Plasmid pBR322 wird die für das CUSI-I-Protein codierende mRNA isoliert. Dazu werden 250 $\mu$g Gesamt-RNA aus menschlichem Cervixgewebe elektrophoretisch in einem denaturierenden, 1,4prozentigen "Low Melting Point" (LMP) Agarosegel, das 15 mM Methylquecksilberhydroxid enthält, der Größe nach aufgetrennt (vgl. Abschnitt 4). Durch Extraktion aus diesem Gel werden ca. 10 $\mu$g mRNA einer Länge von etwa 700 bis 850 Basen isoliert. Von dieser mRNA werden 4 $\mu$g zur cDNA-Synthese (vgl. Abschnitt 7) eingesetzt und in E. coli K12 DH 1 eingeschleust (vgl. Abschnitt 9). Es werden 4300 Transformanten mit 53 ng doppelsträngiger cDNA erhalten. Zur Analyse der Transformanten wird eine Koloniehybridisierung mit 5'-markiertem Oligonucleotid RH5 (vgl. Beispiel 2a) durchgeführt. Die Aktivität des Oligonucleotids in der Hybridisierungslösung beträgt $2 \times 10^5$ cpm/ml bei einer spezifischen Aktivität von 0,72 $\mu$Ci/pmol. Es werden 12 Transformanten isoliert, deren rekombinantes Plasmid mit dem Oligonucleotid RH5 hybridisiert. Ausgehend von den Dauerkulturen der Transformanten werden die rekombinanten Plasmide präpariert und mit Hilfe von Restriktionsspaltungen mit PstI, BamHI und HindIII kartiert. Es stellt sich heraus, daß elf dieser Plasmide ein mit dem Plasmid pRH 31 nahezu identisches Restriktionsmuster aufweisen, d.h. je ein etwa 380 bp und ein etwa 125 bp BamHI-PstI-Fragment sowie je ein etwa 290 bp und ein etwa 200 bp HindIII/PStI-Fragment enthalten. Die Länge der PstI-Fragmente beträgt jeweils etwa 500 bp. Lediglich ein Plasmid weicht in der Restriktionskartierung ab und besitzt ein 285 bp und ein 275 bp BamHI/PstI-Fragment, sowie ein etwa 450 bp und ein etwa 105 bp HindIII/PstI-Fragment. Die Länge der Insertion des abweichenden rekombinanten Plasmids beträgt etwa 550 bp. Dieses rekombinante Plasmid wird pRH 34 genannt.

Seine Restriktionskarte ist in Figur 8 dargestellt. Das Plasmid pRH 34 ist bei der DSM unter der Hinterlegungsnummer DSM 3635 hinterlegt.

c) Nucleotidsequenz der Insertion des rekombinanten Plasmids pRH 34

Das PstI-cDNA-Fragment aus pRH 34 und beide BamHI/PstI-Fragmente aus pRH 34 werden in PstI-bzw. PstI und BamHI-gespaltene und mit alkalischer Phosphatase behandelte DNA des Plasmids pUC18 umkloniert. Die so konstruierten rekombinanten Plasmide, die Hilfskonstruktionen zur DNA-Sequenzanalyse darstellen, tragen die Bezeichnung pRH 1807 (PstI-Fragment), pRH 1808 (N-terminales BamHI/PstI-Fragment) und pRH 1809 (C-terminales BamHI/PstI-Fragment). Die Sequenzanalyse der subclonierten DNA-Fragmente erfolgt nach Maxam und Gilbert (22). Die Sequenzierungsstrategie der rekombinanten Plasmide ist aus Figur 6 ersichtlich.

Die Nucleotidsequenz des cDNA-Fragmentes aus pRH 34 ist in Figur 5 abgebildet. Sie stimmt mit der Sequenz der cDNA-Insertion von pRH 31 von Position 25 bis 308 überein (vgl. Figur 4). Die cDNA-Insertion von pRH 34 ist am 5'-Ende jedoch um 184 bp länger, wobei 143 bp der zur mRNA komplementären Sequenz entsprechen und 41 bp aus der Homopolymer-Verlängerung der cDNA mit dCTP einschließlich der PstI-Spaltstelle hervorgehen.

Die Aminosäuren $\overset{1}{S}$er-Gly-Lys-Ser-Phe wurden als N-Terminus des HUSI-I-Inhibitors identifiziert. Leitet man nun von den 5'-terminal erscheinenden Nucleotiden der isolierten DNA-Sequenz die Aminosäure-Codons ab, erscheint kein Stop-Codon in diesem Bereich des Leserasters. Somit ist das ATG, welches im Leseraster auftaucht, für die Initiation verantwortlich und codiert für den Start des CUSI-I-Proteins. Ferner sind Signalpeptid-Strukturen von sekretorischen Proteinen seiten länger als 25 Aminosäuren. Die Spaltstellen zwischen Signalpeptiden und den entsprechenden natürlich vorkommenden Proteinen befinden sich am häufigsten hinter den Aminosäuren Alanin, Serin und Glycin; die Folge von Gly$\overset{*}{-}$Ser ist also üblich.

Die Primärstruktur des menschlichen CUSI-I-Proteins aus Cervixsekret setzt sich somit aus 107 Aminosäuren zusammen. Die von der ermittelten Nucleotidsequenz codierte Aminosäuresequenz stimmt im wesentlichen mit der bisher nur lückenhaft bekannten N-terminalen Aminosäuresequenz der bronchialen Antileukoprotease überein (41).

**Beispiel 3**

Expression des CUSI-I-Proteins in E. coli

a) Konstruktion eines Expressionsplasmids, das die gesamte CUSI-I-cDNA hinter dem regulierbaren lambda-$p_L$-Promotor enthält.

Als erstes werden zwei synthetische Oligonucleotide synthetisiert, die als RH6 und RH7 bezeichnet werden. Sie sind komplementär zueinander und tragen die Sequenzen für eine optimale Ribosomenbindungsstelle (42), eine SalI- und EcoRI-Spaltstelle und die Nucleotidsequenz der codierenden Region von CUSI-I von Position 1 bis Position 14. Beide Oligonucleotide werden 5'-terminal mit Hilfe des Enzyms T4-Polynucleotid-Kinase phosphoryliert, präparativ in einem 12prozentigen Polyacrylamidgel elektrophoretisch getrennt, aus dem Gel eluiert und an DE 52® chromatographiert. Nach anschließender Gelpermeationschromatographie an Sephadex® G-50 werden 10 pmol jedes Oligonucleotids vermischt, bei 90°C denaturiert und durch langsames Abkühlen auf Raumtemperatur miteinander hybridisiert. Auf diese Weise erhält man das folgende doppelsträngige DNA-Fragment:

```
                                      Met   Lys   Ser   Ser
                    10                    20                    30
RH6 5' A A T T C G G A G G T G T C G A C T A T G A A G T C C A G C G G  3'
RH7           G C C T C C A C A G C T G A T A C T T C A G G T C G C C
      Eco RI          SD            Sal I                        HaeIII
```

Als zweite Komponente wird das für den weiteren N-terminalen Bereich codierende 210 bp (HaeIII/Bam HI) Fragment des Plasmids pRH 34 isoliert. Hierzu werden 10 $\mu$g des Plasmids pRH 34 mit den Restriktionsendonucleasen HaeIII und Bam HI gespalten und auf einem 5prozentigen Polyacrylamidgel elektrophoretisch getrennt. Anschließend wird das 210 bp-Fragment aus dem Polyacrylamidgel eluiert. Als dritte Komponente und somit als Vektor (Figur 7) dient das Plasmid pRH 1807 (Figur 6). Dazu werden 10 $\mu$g dieses Plasmids mit den Restriktionsenzymen EcoRI und BamHI geschnitten. Das Vektor-Fragment wird mit alkalischer Phosphatase behandelt, anschließend mit Phenol extrahiert und mit Äthanol ausgefällt. Für die 3-Komponenten-Ligierung (Figur 7) werden 1 pmol der miteinander hybridisierten synthetischen Oligonucleotide, 0,2 pmol des N-terminalen HaeIII-BamHI-Fragmentes und 0,03 pmol der Vektor-DNA vermischt und in einem Reaktionsvolumen von 30 $\mu$l mit 5U T4-DNA-Ligase miteinander ligiert. Zur Transformation wird der E. coli K12-Stamm JM 101 eingesetzt. Die Plasmide der so erhaltenen Transformanten werden durch Hybridisierung mit radioaktiv markiertem Oligonucleotid RH6 untersucht. Ferner werden die Restriktionsschnittstellen für SalI, EcoRI und BamHI überprüft und die Länge der entsprechenden Fragmente bestimmt. Das richtige neukonstruierte Plasmid wird als pRH 1810 (DSM 3905) bezeichnet (Figur 7).

Zur Konstruktion eines Expressionsplasmids werden 10 $\mu$g des Vektors pWH701 (26) mit den Enzymen EcoRI und SphI geschnitten, dephosphoryliert, mit Phenol extrahiert und mit Äthanol ausgefällt. Zur Präparation des EcoRI-SphI-Fragmentes aus dem Plasmid pRH1810 werden 10 $\mu$g DNA mit den Enzymen EcoRI und SphI geschnitten, das entstehende 525 bp-Fragment durch Gelelektrophorese mit einem 5prozentigem Polyacrylamidgel vom Vektor abgetrennt und aus dem Gel eluiert. Nachfolgend werden 0,3 pmol Vektor pWH 701 und 1 pmol des EcoRI-SphI-Fragmentes miteinander ligiert und in E.coli K12 Wildtyp W6 eingeschleust. Die Plasmide der erhaltenen Transformanten werden über Plasmid-Schnellanalysen und nachfolgende Agarose-Gelelektrophorese charakterisiert. Die rekombinanten Expressionsplasmide sind um 280 bp länger als die Expressionsplasmide ohne Insertion. Das neu identifizierte rekombinierte Expressionsplasmid wird als pRH 24 bezeichnet und für die anschließenden Expressionsversuche verwendet. Figur 7 zeigt das Konstruktionsschema des rekombinanten Expressionsplasmids pRH24.

b) Expression der CUSI-I-cDNA mit dem Expressionsplasmid pRH24 in E. coli K12 MC1061/pRK248 clts (15,27).

Der Wirtsbakterienstamm allein ist nicht lambda-lysogen, das heißt, er enthält keinen lambda-cl-Repressor. Die genetische Information für den thermolabilen Repressor lambda-cl 857 ist auf dem Plasmid pRK248 clts lokalisiert, das dem Wirtsbakterium ferner eine Tetracyclin-Resistenz verleiht (27). Bei 30°C wird die lambda-$p_L$-Promotor gesteuerte Transkription vollständig gehemmt. Bei 42°C liegt der thermolabile cI 857-Repressor in seiner inaktiven Form vor und die hinter stromabwärts dem $p_L$-Promotor liegenden Gene werden transkribiert.

Dieser E. coli K12 MC1061/pRK248 cIts wird mit dem rekombinierten Expressionsplasmid pRH24 transformiert. Zur Induktion der Expression des hinter dem lambda-$p_L$-Promotor liegenden CUSI-I-Gens werden 200 ml LB-Medium (20 $\mu$g/ml Tetracyclin, 50 $\mu$g/ml Ampicillin) mit 3 ml einer Übernachtkultur von E. coli K12 MC1061 pRK248 cIts/pRH24 angeimpft und bei 28°C bis zu einer Zelldichte von 0,7 $A_{578}$-Einheiten/ml kultiviert. Anschließend wird die Kultur bei 42°C weitergeschüttelt. Um Proteinanalysen durchzuführen, werden vor der Induktion der Expression und zu verschiedenen Zeitpunkten nach dem Induktionsstart Zellproben entnommen, je 1 $A_{578}$-Einheit Zellen (etwa 1 x 10$^9$ Zellen) abgefüllt, 3 min bei 12 000 g zentrifugiert und das Zellsediment bei -20°C bis zur Aufarbeitung eingefroren.

**Beispiel 4**

Charakterisierung des Proteinrohextraktes nach Expression des CUSI-I-Proteins in E. coli K12 MC1061/pRK248 cIts/pRH 24

a) Test auf immunologische Kreuzreaktion mit Kaninchen-anti-HUSI-I-Antiserum

Die Zellsedimente werden in 60 $\mu$l Aufschlußpuffer (50 mM Tris-HCl, pH 8,0, 1 mM EDTA, 2 % Triton®-X-100) resuspendiert und mit 40 $\mu$l Reduktionspuffer (4 % SDS, 40 % $\beta$-Mercaptoäthanol, 20 % Glycerin, 0,1 % Bromphenolblau) versetzt. Anschließend werden die Proben 5 min bei 100°C, dann 5 min im Ultraschallbad bei Raumtemperatur und nochmals 5 min bei 100°C inkubiert. 20 $\mu$l des Zellaufschlußvolumens werden in 13,5prozentigem SDS-Polyacrylamid elektrophoretisch getrennt und zum immunologischen Nachweis auf Nitrocellulose transferiert. Der Nitrocellulose-Filter wird 1 h bei 37°C mit "Blocking"-Puffer (50 mM Tris-HCl, pH 7,4, 200 mM NaCl, 0,05 % Tween®-20, 1,5 % Gelatine) inkubiert, um unspezifische Bindungsstellen auf dem Filter abzusättigen. Kaninchen-anti-HUSI-I-Antiserum (1:600 in "Blocking"-Puffer verdünnt) wird zur Erst-Antikörperreaktion eingesetzt (Inkubation: 2 h bei Raumtemperatur), während als Zweit-Antikörper Schaf-anti-Kaninchen-IgG-Peroxidase-Konjugat verwendet wird. Die Substratreaktion der Meerrettich-Peroxidase wird mit Diaminobenzidin durchgeführt.

b) Test auf inhibitorische Aktivität des in E. coli exprimierten Proteinase-Inhibitors

Es werden induzierte (6h nach Induktionsstart) und nicht induzierte Zellproben aufgeschlossen und getestet. Die Zellsedimente (1 $A_{578}$-Einheit Zellen) werden in 50 $\mu$l Lysozym-Aufschlußpuffer (50 mM Tris-HCl, pH 8,0, 1 mM EDTA, 1 mg/ml Lysozym) suspendiert, 10 min bei Raumtemperatur inkubiert, mit 150 $\mu$l 50 mM Tris-HCl, pH 8,0 1 mM EDTA verdünnt und 5 min im Ultraschallbad bei Raumterperatur inkubiert. 20 $\mu$l bzw. 80 $\mu$l des Rohextraktes von nicht induzierten Zellen und induzierten Zellen werden in einem Chymotrypsin-Hemmtest (39) auf inhibitorische Aktivität getestet. Die Ergebnisse sind in Tabelle I zusammengefaßt.

Tabelle I

| Bestimmung der inhibitorischen Aktivität des in E. coli exprimierten CUSI-I-Proteins | | | |
|---|---|---|---|
| Inhibitor-Testlösung E. coli-Extrakt $\mu$l | ind. | nicht ind. | Chymotrypsin-Aktivität von 2 pmol Enzym % |
| - | - | - | 100 |
| 20 | - | + | 99,2 |
| 80 | - | + | 89,7 |
| 20 | + | - | 91,2 |
| 80 | + | - | 56,4 |

Im Vergleich zu der Aktivität von Chymotrypsin nicht induziertem Rohextrakt aus E. coli ist die Hemmung durch Zellextrakte von induzierten Zellen um 8 bzw. 37 % höher.

16

**Beispiel 5**

Expression der C-terminalen CUSI-I-Domäne als $\beta$-Galaktosidase-Fusionsprotein in E. coli K12 JM 101

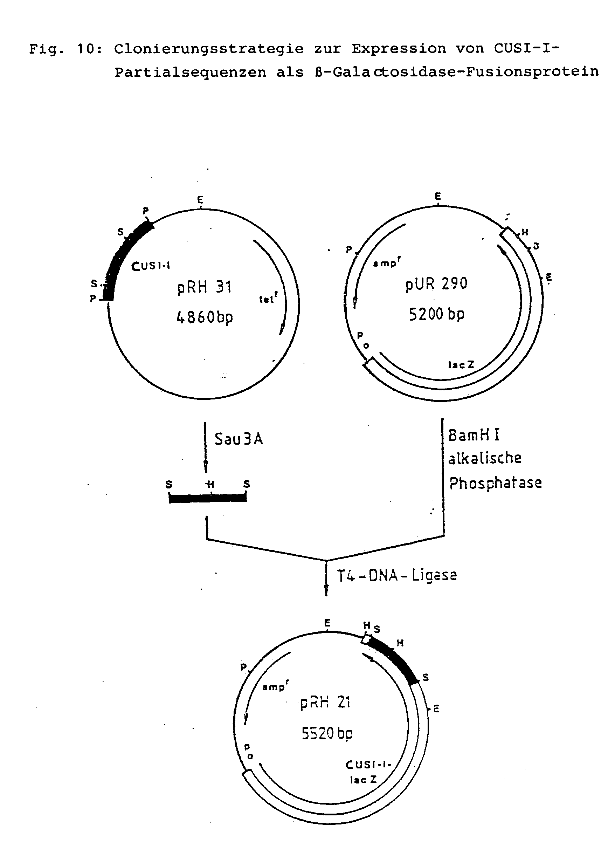

Wie aus Figur 9 hervorgeht, läßt sich die Struktur des CUSI-Inhibitors als ein Proteinmolekül beschreiben, das aus zwei Domänen besteht, die durch eine intragene Duplikation entstanden sind. Um die C-terminale Domäne in E. coli zu exprimieren, wird die für die C-terminalen 59 Aminosäuren codierende DNA-Sequenz im Leseraster mit der den C-Terminus der $\beta$-Galactosidase codierenden DNA-Sequenz des Plasmids pUR 290 (25) verknüpft. Das Schema der Clonierung ist in Figur 10 dargestellt.

10 $\mu$g des Plasmids pRH 31 werden mit der Restriktionsendonuclease Sau3A gespalten, präparativ im 5prozentigen Polyacrylamidgel getrennt und das 320 bp-CUSI-I-Teilfragment aus dem Gel eluiert. pUR 290 (10 $\mu$g) wird mit dem Enzym BamHI gespalten, mit alkalischer Phosphatase behandelt und mit dem 320 bp-Fragment ligiert. Ein Drittel der ligierten DNA wird in CaCl$_2$-behandelte E. coli K12 JM 101 Zellen eingeschleust. Die Selektion der Clone erfolgt auf LB-Amp-Agarplatten (10 g/l Casein enzymatisch gespalten (Sigma), 8 g/l NaCl, 5 g/l Hefeextrakt (Sigma) pH 7,5, 100 $\mu$g/ml Ampicillin). Da zwei Orientierungen des CUSI-I-Teilfragmentes in dem neukombinier-, ten Plasmid möglich sind, werden Plasmid-Schnellanalysen mit anschließender Hind III-Restriktion durchgeführt. Es werden die Clone weiter untersucht, deren Plasmide ein 165 bp-Hind III-Fragment enthalten. Eines dieser Plasmide wird als pRH 21 bezeichnet. Zur Induktion der Fusionsprotein-Expression werden 100 mg LB-Medium, mit 100 $\mu$g/ml Ampicillin aus einer Übernachtkultur mit E. coli K12 JM 101 Bakterien, die mit dem Plasmid pRH 21 transformiert sind ,angeimpft. Die Kultur wird bei 37°C inkubiert und das Wachstum der Zellen bei 578 nm verfolgt. Bei einer optischen Dichte (578 nm) von 0,5 werden die Kulturen auf 500 $\mu$M IPTG eingestellt und bei 37°C weiterinkubiert. Der Verlauf der Induktion des Fusionsproteins wird in Abhängigkeit von der Zeit verfolgt und anhand einer SDS-Polyacrylamid -Gelektrophorese analysiert. Nach der Induktion wird zunächst ausschließlich das CUSI-I-$\beta$-Galaktosidase-Fusionsprotein gebildet, später auch $\beta$-Galactosidase. Um eine immunologische Reaktion mit anti-CUSI-I-Antikörpern nachzuweisen, werden SDS-gelelektrophoretisch getrennte Proteine des E. coli-Rohextraktes auf Nitrocellulose übertragen. Die Western-Blot-Analyse zum spezifischen Nachweis des Fusionsproteins wird wie in Beispiel 4a) beschrieben durchgeführt. Die Isolierung und Reinigung des Fusionsproteins wird wie folgt durchgeführt:

Das IPTG-Analogon TPEG wird nach (37) an CH-Sepharose® gebunden. Zur Reinigung des CUSI-I-$\beta$-Galaktosidase-Fusionsproteins wird der Stamm E. coli K12 JM 101, der das Plasmid pRH 21 enthält, in 1 l LB-Medium, mit 50 $\mu$g/ml Ampicillin kultiviert und bei einer Zelldichte von 0,5 A$_{578}$-Einheiten/ml durch Einstellen des Mediums auf 0,5 mM IPTG induziert. Nach einer Stunde wird die Induktionsphase durch schnelles Abkühlen der Kultur auf 4°C gestoppt. Die Zellen werden sedimentiert (5,5 g Feuchtgewicht), im Lysepuffer suspendiert (20 mM Tris-HCl pH 7,4, 20 mM MgCl$_2$, 20 mM $\beta$-Mercaptoäthanol) und durch Ultraschall-Behandlung aufgeschlossen. Der Rohextrakt wird auf ca. 20 mg/ml Proteinkonzentration und 1,6 M NaCl eingestellt und an TPEG-Sepharose® chromatographiert. Nach dem Waschen des Säulenmaterials mit 20 mM Tris-HCl, pH 7,4, 10 mM $\beta$-Mercaptoäthanol, 10 mM MgCl$_2$, 1,6 M NaCl wird das CUSI-I-$\beta$-Galactosidase-Fusionsprotein mit 100 mM Na-Borat, 10 mM $\beta$-Mercaptoäthanol, pH 10 eluiert. Die Chromatographie wurde mit Hilfe der Bestimmung der $\beta$-Galactosidase-Aktivität verfolgt (43). Aus der 1 l-Kultur konnten 8 mg CUSI-I-$\beta$-Galactosidase rein (90 %) dargestellt werden. Ferner wurde gezeigt, daß das gereinigte Fusionsprotein ebenfalls mit anti-HUSI-I-Antikörpern reagiert.

Zur Abspaltung der C-terminalen CUSI-I-Domäne wird das so gereinigte Fusionsprotein in 10 oder 30%iger Essigsäure oder 70prozentiger Ameisensäure gelöst. Das Vorhandensein einer säurelabilen Asparaginsäure-Prolin-Bindung (vgl. Aminosäuresequenz von CUSI-I) führt nach einer Reaktionszeit von 24 -36 Stunden bei Raumtemperatur zu einer 40- bis 60%igen Abspaltung der C-terminalen CUSI-I-Domäne. Diese intakte CUSI-I-Domäne kann nach dieser Säurebehandlung durch eine Gelfiltration über Sephadex$^R$ G-75 abgetrennt und gereinigt werden. Die Aminosäuresequenz der exprimierten C-terminalen CUSI-I-Domäne lautet:

```
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH.
```

Diese Sequenz ist identisch mit den Positionen 50 - 107 des gesamten CUSI-I-Proteins.

**Beispiel 6**

Expression der C-terminalen CUSI-I-Domäne in E. coli K12JM101

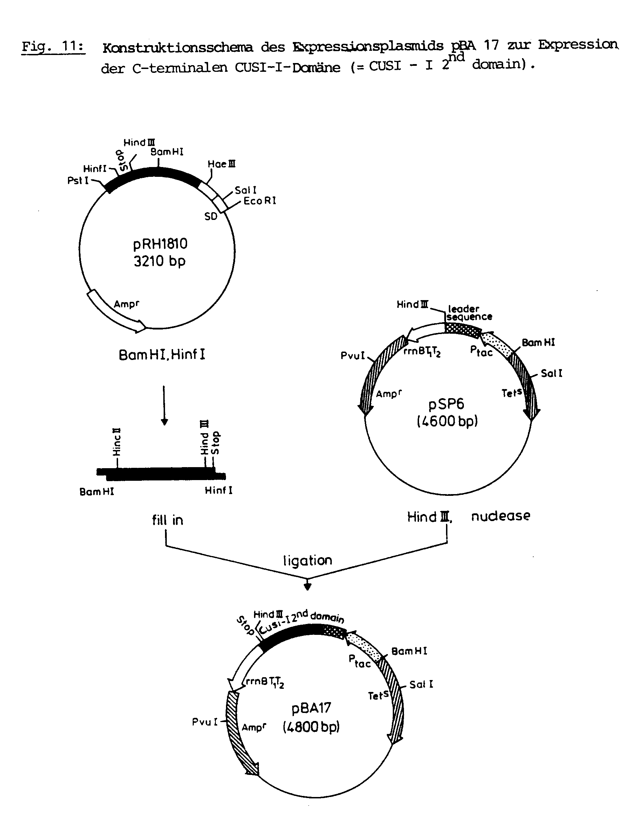

Wie aus Beispiel 5 hervorgeht, ist die C-terminale CUSI-I-Domäne als $\beta$-Galaktosidase-Fusionsprotein in E. coli K12JM101 exprimierbar. Um die C-terminale CUSI-I-Domäne als natives Protein zu exprimieren,wird, wie in Figur 11 gezeigt, die die C-terminalen 59 Aminosäuren codierende cDNA mit der Signalsequenz des Gens der alkalischen Phosphatase des Plasmids pSP6 im Leseraster fusioniert. Das Plasmid pSP6 ist bei der Deutsche Sammlung für Mikroorganismen hinterlegt unter der Hinterlegungsnummer DSM 3904.

30 $\mu$g des Plasmids pRH1810 werden mit BamHI und HinfI gespalten. Die Auftrennung der DNA-Fragmente erfolgt in einem 8% Polyacrylamidgel. Das 175 bp CUSI-I-DNA-Fragment, das für die C-terminale CUSI-I-Domäne codiert, wird aus dem Gel eluiert. Die überstehenden DNA-Enden wurden mit Hilfe des Klenow-Fragments der DNA-Polymerase aufgefüllt. Dabei entsteht ein 182 bp langes doppelsträngiges DNA-Molekül mit glatten Enden.

3 $\mu$g des Vektors pSP6 werden mit der Restriktionsendonuclease HindIII gespalten. Die überstehenden einzelsträngigen DNA-Enden werden mit "Mungbean" (Mungobohnen)-Nuclease abgebaut und die 5'-terminalen Phosphatrestewerden mit alkalischer Phosphatase entfernt.

0,3 pMol des so behandelten Vektors werden mit 1 pMol des C-terminalen CUSI-I-DNA-Fragments ligiert. Mit der Hälfte der erhaltenen Ligierungsprodukte werden transformationskompetente (17) Zellen des Stammes E. coli K12 DH1 transformiert. Anschließend werden Clone auf LB-Amp-Agarplatten selektiert. Durch Koloniehybridisierung mit dem Oligonucleotid AH 12 werden 4 Clone identifiziert, deren Plasmid-DNA zum verwendeten Sondenmolekül komplementäre DNA-Sequenzen enthält . Das Oligonucleotid AH12 ist komplementär zur Sequenz der Nucleotide 241 - 258 des CUSI-I-cDNA-Clons und hat die Sequenz 5'CCT GTT GAC ACC CCA AAC3'.

Durch Plasmidschnellanalyse und Spaltung der DNA mit HindIII und BamHI wird ein Clon identifiziert, der ein 540 bp großes DNA-Fragment als Insertion enthält. Dieses DNA-Fragment besteht aus der Promotorregion $P_{tac}$, der Signalpeptid-codierenden Sequenz des Gens der alkalischen Phosphatase und der cDNA-Sequenz für die C-terminale CUSI-I-Domäne. Das so konstruierte Plasmid erhält die Bezeichnung pBA 17.

Kompetente E. coli K12 JM 101 Zellen werden sodann mit DNA des konstruierten Expressionsplasmids pBA 17 transformiert. Zur Expression der C-terminalen CUSI-I-Domäne werden 250 ml LB-Amp-Medium mit den erhaltenen Transformanten angeimpft. Sodann wird bei 37°C inkubiert. Das Wachstum der Zellen wird durch Messung der Trübung bei 578 nm verfolgt. Bei einer optischen Dichte von 0,97 erfolgt die Zugabe von IPTG in einer Endkonzentration von 0,5 mM, um die Expression zu induzieren. Je 1 $A_{578}$-Einheit Zellen wird zu verschiedenen Zeitpunkten vor und nach der Induktion der Expression entnommen (siehe Tabelle II unten), 3 min bei 12000 g zentrifugiert und das Zellsediment wird bei -20°C gelagert. Das Expressionsprodukt umfasst 59 Aminosäuren mit der Sequenz

```
Asp-Pro-Val-Asp-Thr-Pro-Asn-Pro-Thr-Arg-Arg-Lys-Pro-Gly-Lys-Cys-Pro-Val-

Thr-Tyr-Gly-Gln-Cys-Leu-Met-Leu-Asn-Pro-Pro-Asn-Phe-Cys-Glu-Met-Asp-

Gly-Gln-Cys-Lys-Arg-Asp-Leu-Lys-Cys-Cys-Met-Gly-Met-Cys-Gly-Lys-Ser-

Cys-Val-Ser-Pro-Val-Lys-Ala-OH.
```

Test auf inhibitorische Aktivität der in E. coli exprimierten C-terminalen CUSI-I-Domäne

Der Chymotrypsin-Hemmtest (39) wird wie im Beispiel 4 beschrieben durchgeführt. Die Zunahme der inhibitorischen Aktivität in E. coli-Rohextrakt gegenüber Chymotrypsin wird in Abhängigkeit von der Zeit verfolgt. Jeweils die Hälfte (100 $\mu$l) des E. coli-Lysats wird dabei auf inhibitorische Aktivität getestet. Die Ergebnisse sind in Tabelle II zusammengefaßt.

EP 0 232 523 B1

T a b e l l e    II

Bestimmung der inhibitorischen Aktivität der in E. coli exprimierten C-terminalen CUSI-I-Domäne

| Probeentnahme | m IU/ml Kultur | Chymotrypsin-Aktivität von 2 pMol Enzym (%) |
|---|---|---|
| vor Induktion (Stunden) | | |
| 2 | 0,624 | 90 |
| 2,5 | 1,88 | 75 |
| 3 | 2,00 | 77 |
| nach Induktion (Stunden) | | |
| 1 | 4,83 | 67 |
| 2 | 6,57 | 67 |
| 3 | 7,24 | 67 |
| 4 | 9,56 | 64 |
| 5 | 8,00 | 67 |
| 6 | 10,66 | 60 |
| 21 | 16,96 | 37 |

In Tabelle III sind die nach dem Budapester Vertrag hinterlegten Mikroorganismen zusammengefaßt.

Tabelle III

| Mikroorganismus | Hinterlegungsstelle | Hinterlegungsnummer |
|---|---|---|
| E.coli K12 MC1061 | DSM | 3631 |
| E.coli K12 JM101 | ATCC | 33876 |
| E.coli K12 DH1 | ATCC | 33849 |
| E.coli K12 W6 | DSM | 3632 |
| pBR 322 | ATCC | 31344 |
| pUC 18 | DSM | 3424 |
| pUR 290 | DSM | 3417 |
| pWH 701 | DSM | 3633 |
| pRK 248clts | ATCC | 33766 |
| pRH 31 | DSM | 3634 |
| pRH 34 | DSM | 3635 |
| pSP6 | DSM | 3904 |
| pRH 1810 | DSM | 3905 |

Literaturverzeichnis

1. Rodmer et al., Schweiz. med. Wschr. 144, 1359 - 1363 (1984)
2. Lewis, D.A., Biochem. Pharmacol. 33, 1705 - 1714 (1984)
3. Schiessler, H. et al., Bayer Symp. V, Proteinase Inhihitors (Fritz, H., Tschesche, H., Greene, L.J., Truscheit, E., Hrsg.) pp. 147 - 155, Springer Verlag, Berlin (1974)
4. Wallner, O. und Fritz, H., Hoppe-Seyler's Z. Physiol. Chem. 355, 709 - 715 (1974)

5. Fritz, H. et al. (1975) in: Proteases and Biological Control (Reich, F. Rifkin, D. B. und Shaw, E., Hrsg.), 737 - 766, Cold Spring Harbor Laboratory

6. Wallner, O. et al. in: Protides of the Riological Fluids (Peters, H., ed.) 23, 177 - 182, Pergamon Press, Oxford (1975)

7. Chirgwin, J.M., et al., Biochemistry, 18, 5294-5299 (1979)

8. Thayer, R. E., Anal. Biochem. 98, 60 - 63 (1979)

9. Wallace, R. B. et al. Nucl. Acids Res. 9, 879 - 894 (1981)

10. Clark, L. et al., Meth. Enzymol. 68, 436 - 442 (1979)

11. Gershoni, J.M. und Palade, G. E., Anal. Biochem. 131, 1-15 (1983)

12. Towbin, H. et al., Proc. Natl. Acad. Sci. USA 76, 4350 - 4354 (1979)

13. Guhler, U. und Hoffman, B. J., Gene 25, 263 - 269 (1983)

14. Volkaert, G. et al. in: Advanced Molecular Genetics, Springer Verlag, Berlin, 255 - 257 (1984)

15. Casadaban, M.J. und Cohen, S.N. (1980), J. Mol. Biol., 138, 179-207

16. Messing, J. et al., Nucl. Acids Res. 9, 309-321 (1981)

17. Hanahan, D., J. Mol. Biol. 166, 557 - 580 (1983)

19. McDonnell, M. W. et al., J. Mol. Biol 110, 119 (1977)

20. Bailey, J.M. und Davidson, N., Anal. Biochem. 70, 75 - 85 (1976)

21. Laemmli, U. K., Nature 227, 680 - 685 (1970)

22. Maxam, A. M., und Gilbert, W., Meth. Enzymol. 65, 499 - 580 (1980)

23. Bolivar, F. et al., Gene 2, 95-113 (1977)

24. Yanish-Perron, C. et al., Gene 33, 103 - 119 (1985)

25. Rüther, U., Müller-Hill, B., EMBO-J. 2, 1791 - 1794 (1983)

26. C. Gatz, TH Darmstadt, Dissertation, 89-93 (1985)

27. Bernard, H.U. et al., Gene 5, 59-76 (1979)

28. Maniatis, T. et al.: Molecular cloning: A laboratory manual, Gold Spring Harbor Laboratory, Cold Spring Harbor, New York, 194 (1982); Bonner, T.I. et al., J. Mol. Biol. 81, 123 (1973)

29. Glisin, V. et al., Biochemistry 13, 2633 (1974)

30. Aviv, H. und Leder, P., Proc. Natl. Acad. Sci. USA 69, 1408 (1972)

31. Thomas, P. S., Proc. Natl. Acad. Sci. USA 77, 5201 (1980)

32. Suggs, S.V. et al., Developmental biology using purified genes (D. Brown, ed.) Academic Press, New York, 683 (1981)

33. Mandel, M. und Higa, A., J. Mol. Biol. 53, 154 - 162 (1970)

34. Hardies, S. C. et al., J. Biol. Dhem. 254, 5527 - 5534 (1979)

35. Holmes, D. S. und Ouigley, M., Anal. Biochem. 114, 193 - 197 (1981)

36. Caruthers, M.H., in Chemical and Enzymatic Gene Synthesis (H. G. Gassen, A. Lang, Hrsg.), 1. Aufl., Verlag Chemie, Weinheim (1982).

37. Ullmann, A. Gene 29, 27 - 31 (1984)

38. Fritz, H. et al.: Methoden der enzymatischen Analyse (Ed. H. U. Bergmeyer), 3. Aufl., 1105, Verlag Chemie, Weinheim (1974)

39. DelMar, F. G. et al., Anal. Biochem. 99, 316 - 320 (1979)

40. Pelham, R. B. und Jackson, R. J., Eur. J. Biochem. 67, 247 - 256 (1976)

41. Klasen, E.C. und Kramps, J.A., Biochem. Biophys. Res. Comm. 128, 285-289 (1985)

42. Guarente, L. et al., Science 209, 1428-1430 (1980)

43. Miller, J. H., Experiments in Molecular Genetics, Cold Spring Harbor Laboratory, Cold Spring Harbor, New York, 433 (1972)

44. Ohlson, K. et al., Hoppe Seyler's Z. Physiol. Chem. 357, 1241 - 1244 (1977)

45. Hochstrasser, K. et al, Hoppe-Seyler's Z. Physiol. Chem. 362, 1369 - 1375 (1981)

46. Smith, C.E. und Johnson, D.A., Biochem. J. 225, 463-472 (1985)

47. Schiessler, H. et al., (1978)
Neutral Proteinases of Human Polymorphnuclear Leukocytes (Havemann, K. und Janoff, A., eds.) 195-207, Urban und Schwarzenberg, Baltimore, München

48. Fritz, H. (1980) in Protein degradation in health and disease, Ciba Foundation Symposium 75, 351-379 publ. Excerpta Medica, Elsvier, North Holland

49. Bernard, H. -U., Methods Enzymol. 68, 482 - 492 (1979).

50. Machleidt, W.,
Modern Methods in Protein Chemistry (Tschesche, H., ed.), 267 - 302, Walter de Gruyter, Berlin, New York (1983)

EP 0 232 523 B1

**Patentansprüche**
**Patentansprüche für folgende Vertragsstaaten : BE, CH, DE, FR, GB, IT, LI, LU, NL, SE**

1. DNA-Sequenz, **dadurch gekennzeichnet**, daß sie ein Protein mit der biologischen Aktivität des CUSI-I-Proteins mit der in Figur 4 dargestellten Aminosäuresequenz codiert.

2. DNA-Sequenz, **dadurch gekennzeichnet**, daß sie ein Protein mit der biologischen Aktivität des CUSI-I-Proteins mit der in Figur 5 dargestellten Aminosäuresequenz codiert.

3. DNA-Sequenz, **dadurch gekennzeichnet**, daß sie ein Protein mit der biologischen Aktivität des CUSI-I-Proteins codiert und die in Figur 4 dargestellte Sequenz ist, die im rekombinanten Plasmid pRH 31 (DSM 3634) als PstI-Fragment enthalten ist.

4. DNA-Sequenz, **dadurch gekennzeichnet**, daß sie ein Protein mit der biologischen Aktivität des CUSI-I-Proteins codiert und die in Figur 5 dargestellte Sequenz ist, die im rekombinanten Plasmid pRH 34 (DSM 3635) als PstI-Fragment enthalten ist.

5. DNA-Fragment mit der Sequenz

5' A A T T C G G A G G T G T C G A C T A T G A A G T C C A G C G G 3'
         G C C T C C A C A G C T G A T A C T T C A G G T C G C C

6. Rekombinanter Vektor zur Clonierung, **dadurch gekennzeichnet**, daß er eine DNA-Sequenz nach einem der Ansprüche 1 bis 5 enthält.

7. Rekombinanter Vektor zur Expression, **dadurch gekennzeichnet**, daß er eine DNA-Sequenz nach einem der Ansprüche 1 bis 5 enthält, die funktionell mit einer Expressionskontroll-Sequenz verbunden ist.

8. Rekombinanter Vektor nach Anspruch 7, **dadurch gekennzeichnet**, daß die Expressionskontroll-Sequenz der E. coli lac-Promotor, der E. coli trp-Promotor, der E. coli Lipoprotein-Promotor, der lambda-PL- oder PR-Promotor, eine Hefe-Expressionskontroll-Sequenz oder eine andere eukaryontische Expressionskontroll-Sequenz ist.

9. Plasmid pRH 31 (DSM 3634).

10. Plasmid pRH 34 (DSM 3635).

11. Plasmid pRH 1810 (DSM 3905).

12. Plasmid pRH 24, erhältlich entsprechend dem Konstruktionsschema gemäß Figur 7 ausgehend vom Plasmid pRH 1810 (DSM 3905).

13. Plasmid pRH 21, erhältlich entsprechend dem Konstruktionsschema gemäß Figur 10 ausgehend vom Plasmid pRH 31 (DSM 3634).

14. Plasmid pBA17, erhältlich entsprechend dem Konstruktionsschema gemäß Figur 11 ausgehend vom Plasmid pRH 1810 (DSM 3905).

15. Wirtsorganismus, **dadurch gekennzeichnet**, daß er mit einem der rekombinanten Vektoren nach Anspruch 6 bis 14 transformiert ist.

16. Wirtsorganismus nach Anspruch 15, **dadurch gekennzeichnet**, daß er ein Stamm der Art E. coli, B. subtilis oder ein anderes Bakterium, Saccharomyces cerevisiae, ein anderer mikroskopisch kleiner Pilz,

21

eine tierische oder eine menschliche Zelle ist.

17. Protein, **dadurch gekennzeichnet**, daß es die biologische Aktivität des CUSI-I-Proteins aufweist und die in Figur 4 dargestellte Aminosäuresequenz hat.

18. Protein, **dadurch gekennzeichnet**, daμ es die biologische Aktivität des CUSI-I-Proteins aufweist und die in Figur 5 dargestellte Aminosäuresequenz hat.

19. Protein, **dadurch gekennzeichnet**, daß es die biologische Aktivität des CUSI-I-Proteins aufweist und die Aminosäuresequenz

Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH

hat.

20. Protein, **dadurch gekennzeichnet**, daß es die biologische Aktivität des CUSI-I-Proteins aufweist und die Aminosäuresequenz

Asp-Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH.

hat.

21. Verfahren zur Herstellung eines Proteins nach einem der Ansprüche 17 bis 20, **dadurch gekennzeichnet**, daß man einen Wirtsorganismus nach Anspruch 15 oder 16 in einem üblichen Nährmedium züchtet, gegebenenfalls die Expression des Genprodukts induziert, das Expressionsprodukt aus der Kultur isoliert und gegebenenfalls einer kontrollierten sauren Hydrolyse unterwirft und das gewünschte biologisch aktive Proteinfragment aus dem Hydrolysat durch Gelchromatographie abtrennt.

22. Arzneimittel, **gekennzeichnet durch** einen Gehalt an einem Protein nach einem der Ansprüche 17 bis 20 und üblichen Trägerstoffen und/oder Verdünnungsmitteln und/oder Hilfsstoffen.

23. Arzneimittel nach Anspruch 22, als Spray oder Inhalationsmittel.

24. Arzneimittel nach Anspruch 22 oder 23 zur Behandlung von chronischer Bronchitis, von chronischen Cervixentzündungen, von mit exzessiver Schleimbildung verbundenen chronisch-entzündlichen Prozessen und den sich daraus ergebenden Notsituationen, sowie von postoperativen Blutungen aufgrund einer Hyperfibrinolyse und zur Frühtherapie von Schockzuständen.

**Patentansprüche für folgenden Vertragsstaat : GR**

1. DNA-Sequenz, **dadurch gekennzeichnet**, daß sie ein Protein mit der biologischen Aktivität des CUSI-I-Proteins mit der in Figur 4 dargestellten Aminosäuresequenz codiert.

2. DNA-Sequenz, **dadurch gekennzeichnet**, daß sie ein Protein mit der biologischen Aktivität des CUSI-I-Proteins mit der in Figur 5 dargestellten Aminosäuresequenz codiert.

**3.** DNA-Sequenz, **dadurch gekennzeichnet**, daß sie ein Protein mit der biologischen Aktivität des CUSI-I-Proteins codiert und die in Figur 4 dargestellte Sequenz ist, die im rekombinanten Plasmid pRH 31 (DSM 3634) als PstI-Fragment enthalten ist.

**4.** DNA-Sequenz, **dadurch gekennzeichnet**, daß sie ein Protein mit der biologischen Aktivität des CUSI-I-Proteins codiert und die in Figur 5 dargestellte Sequenz ist, die im rekombinanten Plasmid pRH 34 (DSM 3635) als PstI-Fragment enthalten ist.

**5.** DNA-Fragment mit der Sequenz

```
5' A A T T C G G A G G T G T C G A C T A T G A A G T C C A G C G G 3'
      G C C T C C A C A G C T G A T A C T T C A G G T C G C C
```

**6.** Rekombinanter Vektor zur Clonierung, **dadurch gekennzeichnet**, daß er eine DNA-Sequenz nach einem der Ansprüche 1 bis 5 enthält.

**7.** Rekombinanter Vektor zur Expression, **dadurch gekennzeichnet**, daß er eine DNA-Sequenz nach einem der Ansprüche 1 bis 5 enthält, die funktionell mit einer Expressionskontroll-Sequenz verbunden ist.

**8.** Rekombinanter Vektor nach Anspruch 7, **dadurch gekennzeichnet**, daß die Expressionskontroll-Sequenz der E. coli lac-Promotor, der E. coli trp-Promotor, der E. coli Lipoprotein-Promotor, der lambda-PL- oder PR-Promotor, eine Hefe-Expressionskontroll-Sequenz oder eine andere eukaryontische Expressionskontroll-Sequenz ist.

**9.** Plasmid pRH 31 (DSM 3634).

**10.** Plasmid pRH 34 (DSM 3635).

**11.** Plasmid pRH 1810 (DSM 3905).

**12.** Plasmid pRH 24, erhältlich entsprechend dem Konstruktionsschema gemäß Figur 7 ausgehend vom Plasmid pRH 1810 (DSM 3905).

**13.** Plasmid pRH 21, erhältlich entsprechend dem Konstruktionsschema gemäß Figur 10 ausgehend vom Plasmid pRH 31 (DSM 3634).

**14.** Plasmid pBA17, erhältlich entsprechend dem Konstruktionsschema gemäß Figur 11 ausgehend vom Plasmid pRH 1810 (DSM 3905).

**15.** Wirtsorganismus, **dadurch gekennzeichnet**, daß er mit einem der rekombinanten Vektoren nach Anspruch 6 bis 14 transformiert ist.

**16.** Wirtsorganismus nach Anspruch 15, **dadurch gekennzeichnet**, daß er ein Stamm der Art E. coli, B. subtilis oder ein anderes Bakterium, Saccharomyces cerevisiae, ein anderer mikroskopisch kleiner Pilz, eine tierische oder eine menschliche Zelle ist.

**17.** Protein, **dadurch gekennzeichnet**, daß es die biologische Aktivität des CUSI-I-Proteins aufweist und die in Figur 4 dargestellte Aminosäuresequenz hat.

**18.** Protein, **dadurch gekennzeichnet**, daß es die biologische Aktivität des CUSI-I-Proteins aufweist und die in Figur 5 dargestellte Aminosäuresequenz hat.

**19.** Protein, **dadurch gekennzeichnet**, daß es die biologische Aktivität des CUSI-I-Proteins aufweist und die Aminosäuresequenz

EP 0 232 523 B1

Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH

hat.

**20.** Protein, **dadurch gekennzeichnet**, daß es die biologische Aktivität des CUSI-I-Proteins aufweist und die Aminosäuresequenz

Asp-Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH.

hat.

**21.** Verfahren zur Herstellung eines Proteins nach eine der Ansprüche 17 bis 20, **dadurch gekennzeichnet**, daß min einen Wirtsorganismus nach Anspruch 15 oder 16 in einem üblichen Nährmedium züchtet, gegebenenfalls die Expression des Genprodukts induziert, das Expressionsprodukt aus der Kultur isoliert und gegebenenfalls einer kontrollierten sauren Hydrolyse unterwirft und das gewünschte biologisch aktive Proteinfragment aus dem Hydrolysat durch Gelchromatographie abtrennt.

**Patentansprüche für folgende Vertragsstaaten : AT, ES**

**1.** Verfahren zur Herstellung einer DNA-Sequenz, die ein Protein mit der biologischen Aktivität des CUSI-I-Proteins mit der in Fig. 4 dargestellten Aminosäuresequenz codiert, **dadurch gekennzeichnet**, daß ein für die Synthese des Proteins codierendes Gen mittels rekombinanter DNA-Technologie identifiziert und isoliert wird.

**2.** Verfahren zur Herstellung einer DNA-Sequenz, die ein Protein mit der biologischen Aktivität des CUSI-I-Proteins mit der in Fig. 5 dargestellten Aminosäuresequenz codiert, dadurch gekennzeichnet, daß ein für die Synthese des Proteins codierendes Gen mittels rekombinanter DNA-Technologie identifiziert und isoliert wird.

**3.** Verfahren zur Herstellung einer DNA-Sequenz nach Anspruch 1, **dadurch gekennzeichnet**, daß die DNA-Sequenz im rekombinanten Plasmid pRH 31 (DSM 3634) als PstI-Fragment enthalten ist.

**4.** Verfahren zur Herstellung einer DNA-Sequenz nach Anspruch 2, **dadurch gekennzeichnet**, daß die DNA-Sequenz im rekombinanten Plasmid pRH 34 (DSM 3635) als PstI-Fragment enthalten ist.

**5.** Verfahren zur Herstellung eines DNA-Fragments mit der Sequenz

```
5' A A T T C G G A G G T G T C G A C T A T G A A G T C C A G C G G 3'
        G C C T C C A C A G C T G A T A C T T C A G G T C G C C
```

**dadurch gekennzeichnet**, daß zwei synthetische, zueinander komplementäre Oligonucleotide mitein-ander hybridisiert werden.

24

6.   Verfahren zur Herstellung eines rekombinanten Vektors zur Clonierung, **dadurch gekennzeichnet**, daß eine nach einem der Verfahren der Ansprüche 1 bis 5 hergestellte DNA-Sequenz gegebenenfalls nach Ligierung gemäß Fig. 7 in einen üblichen Clonierungsvektor cloniert wird.

7.   Verfahren zur Herstellung eines rekombinanten Vektors zur Expression, **dadurch gekennzeichnet**, daß eine nach einem der Verfahren der Ansprüche 1 bis 5 hergestellte DNA-Sequenz gegebenenfalls nach Ligierung gemäß Fig. 7 so in einen üblichen Expressionsvektor cloniert wird, daß die DNA-Sequenz funktionell mit einer Expressionskontroll-Sequenz verbunden ist.

8.   Verfahren zur Herstellung eines rekombinanten Vektors nach Anspruch 7, **dadurch gekennzeichnet**, daß die Expressions-Kontrollsequenz der E. coli lac-Promotor, der E. coli trp-Promotor, der E.coli Lipoprotein-Promotor, der lambda-PL- oder PR-Promotor, eine Hefe-Expressionskontroll-Sequenz oder eine andere eukaryontische Expressionskontroll-Sequenz ist.

9.   Verfahren zur Herstellung des Plasmids pRH 24, **dadurch gekennzeichnet**, daß entsprechend dem Konstruktionsschema gemäß Fig. 7 ausgehend vom Plasmid pRH 1810 (DSM 3905) verfahren wird.

10.  Verfahren zur Herstellung des Plasmids pRH 21, **dadurch gekennzeichnet**, daß entsprechend dem Konstruktionsschema gemäß Fig. 10 ausgehend vom Plasmid pRH 31 (DSM 3634) verfahren wird.

11.  Verfahren zur Herstellung des Plasmids pBA 17, **dadurch gekennzeichnet**, daß entsprechend dem Konstruktionsschema gemäß Fig. 11 ausgehend vom Plasmid pRH 1810 (DSM 3905) verfahren wird.

12.  Verfahren zur Herstellung eines transformierten Wirtsorganismus, **dadurch gekennzeichnet**, daß ein Wirtsorganismus mit einem der nach einem der Verfahren 6 bis 11 hergestellten Vektoren transformiert wird.

13.  Verfahren zur Herstellung eines transformierten Wirtsorganismus nach Anspruch 12, **dadurch gekennzeichnet**, daß der Wirtsorganismus ein Stamm der Art E. coli, B. subtilis oder ein anderes Bakterium, Saccharomyces cerevisiae, ein anderer mikroskopisch kleiner Pilz, eine tierische oder eine menschliche Zelle ist.

14.  Verfahren zur Herstellung eines Proteins, das die biologische Aktivität des CUSI-I-Proteins aufweist und die in Fig. 4 dargestellte Aminosäuresequenz hat, **dadurch gekennzeichnet**, daß man einen nach einem der Verfahren der Ansprüche 12 oder 13 herstellbaren Wirtsorganismus in einem üblichen Nährmedium züchtet, gegebenenfalls die Expression des Genprodukts induziert, das Expressionsprodukt aus der Kultur isoliert und gegebenenfalls einer kontrollierten sauren Hydrolyse unterwirft und das gewünschte biologisch aktive Proteinfragment aus dem Hydrolysat durch Gelchromatographie abtrennt.

15.  Verfahren zur Herstellung eines Proteins, das die biologische Aktivität des CUSI-I-Proteins aufweist und die in Fig. 5 dargestellte Aminosäuresequenz hat, dadurch gekennzeichnet, daß man wie in Anspruch 14 verfährt.

16.  Verfahren zur Herstellung eines Proteins nach Anspruch 14, das einen Teilbereich der in Fig. 4 dargestellten Aminosäuresequenz umfaßt, dadurch gekennzeichnet, daß das Protein die Aminosäuresequenz

Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH

hat.

17. Verfahren zur Herstellung eines Proteins nach Anspruch 14, das einen Teilbereich der in Fig. 4 dargestellten Aminosäuresequenz umfaßt, **dadurch gekennzeichnet**, daß das Protein die Aminosäuresequenz

Asp-Pro-Val-Asp-Thr-Pro-Asn-Pro-Thr-Arg-Arg-Lys-Pro-Gly-Lys-Cys-Pro-Val-

Thr-Tyr-Gly-Gln-Cys-Leu-Met-Leu-Asn-Pro-Pro-Asn-Phe-Cys-Glu-Met-Asp-

Gly-Gln-Cys-Lys-Arg-Asp-Leu-Lys-Cys-Cys-Met-Gly-Met-Cys-Gly-Lys-Ser-

Cys-Val-Ser-Pro-Val-Lys-Ala-OH.

hat.

18. Verfahren zur Herstellung eines Arzneimittels, **dadurch gekennzeichnet**, daß ein nach einem der Verfahren der Ansprüche 14 bis 17 hergestelltes Protein mit üblichen Trägerstoffen und/oder Verdünnungsmitteln und/oder Hilfsstoffen gemischt wird.

19. Verfahren zur Herstellung eines Arzneimittels nach Anspruch 18, **dadurch gekennzeichnet**, daß das Arzneimittel als Spray oder Inhalationsmittel verwendet werden kann.

20. Verfahren zur Herstellung eines Arzneimittels nach Anspruch 18 oder 19, **dadurch gekennzeichnet**, daß das Arzneimittel zur Behandlung von chronischer Bronchitis, von chronischen Cervixentzündungen, von mit exzessiver Schleimbildung verbundenen chronisch-entzündlichen Prozessen und den sich daraus ergebenden Notsituationen, sowie von postoperativen Blutungen aufgrund von Hyperfibrinolyse und zur Frühtherapie von Schockzuständen verwendet werden kann.

**Claims**
**Claims for the following Contracting States : BE, CH, DE, FR, GB, IT, LI, LU, NL, SE**

1. A DNA sequence, **characterized in that** it encodes a protein having the biological activity of the CUSI-I protein with the amino acid sequence shown in Figure 4.

2. A DNA sequence, **characterized in that** it encodes a protein having the biological activity of the CUSI-I protein with the amino acid sequence shown in Figure 5.

3. A DNA sequence, **characterized in that** it encodes a protein having the biological activity of the CUSI-I protein and is the sequence shown in Figure 4 which is contained in the recombinant plasmid pRH 31 (DSM 3634) as PstI fragment.

4. A DNA sequence, **characterized in that** it encodes a protein having the biological activity of the CUSI-I protein and is the sequence shown in Figure 5 which is contained in the recombinant plasmid pRH 34 (DSM 3635) as PstI fragment.

5. A DNA fragment with the sequence

5' A A T T C G G A G G T G T C G A C T A T G A A G T C C A G C G G 3'
        G C C T C C A C A G C T G A T A C T T C A G G T C G C C

6. A recombinant vector for cloning, **characterized in that** it contains a DNA sequence according to any one of claims 1 to 5.

7. A recombinant vector for expression, **characterized in that** it contains a DNA sequence according to any one of claims 1 to 5 which is operatively linked with an expression control sequence.

8. The recombinant vector according to claim 7, **characterized in that** the expression control sequence is the E. coli lac promoter, the E. coli trp promoter, the E. coli lipoprotein promoter, the lambda-PL or PR promoter, a yeast expression control sequence or another eukaryotic expression control sequence.

9. Plasmid pRH 31 (DSM 3634).

10. Plasmid pRH 34 (DSM 3635).

11. Plasmid pRH 1810 (DSM 3905).

12. Plasmid pRH 24, obtainable according to the construction scheme in Figure 7 starting from plasmid pRH 1810 (DSM 3905).

13. Plasmid pRH 21, obtainable according to the construction scheme in Figure 10 starting from plasmid pRH 31 (DSM 3634).

14. Plasmid pBA17, obtainable according to the construction scheme in Figure 11 starting from plasmid pRH 1810 (DSM 3905).

15. A host organism, **characterized in that** it is transformed with one of the recombinant vectors according to claims 6 to 14.

16. The host organism according to claim 15, **characterized in that** it is a strain of the species E. coli, B. subtilis or another bacterium, Saccharomyces cerevisiae, another microscopically small fungus, an animal or a human cell.

17. A protein, **characterized in that** it has the biological activity of the CUSI-I protein and it has the amino acid sequence shown in Figure 4.

18. A protein, **characterized in that** it has the biological activity of the CUSI-I protein and it has the amino acid sequence shown in Figure 5.

19. A protein, **characterized in that** it has the biological activity of the CUSI-I protein and it has the amino acid sequence

Pro-Val-Asp-Thr-Pro-Asn-Pro-Thr-Arg-Arg-Lys-Pro-Gly-Lys-Cys-Pro-Val-Thr-Tyr-Gly-Gln-Cys-Leu-Met-Leu-Asn-Pro-Pro-Asn-Phe-Cys-Glu-Met-Asp-Gly-Gln-Cys-Lys-Arg-Asp-Leu-Lys-Cys-Cys-Met-Gly-Met-Cys-Gly-Lys-Ser-Cys-Val-Ser-Pro-Val-Lys-Ala-OH

20. A protein, **characterized in that** it has the biological activity of the CUSI-I protein and it has the amino acid sequence

Asp-Pro-Val-Asp-Thr-Pro-Asn-Pro-Thr-Arg-Arg-Lys-Pro-Gly-Lys-Cys-Pro-Val-Thr-Tyr-Gly-Gln-Cys-Leu-Met-Leu-Asn-Pro-Pro-Asn-Phe-Cys-Glu-Met-Asp-Gly-Gln-Cys-Lys-Arg-Asp-Leu-Lys-Cys-Cys-Met-Gly-Met-Cys-Gly-Lys-Ser-Cys-Val-Ser-Pro-Val-Lys-Ala-OH.

21. A method for the preparation of a protein according to any one of claims 17 to 20, **characterized in that** a host organism according to claim 15 or 16 is cultured in a conventional nutrient medium, the

EP 0 232 523 B1

expression of the gene product is optionally induced, the expression product is isolated from the culture and optionally subjected to a controlled acidic hydrolysis and the desired biologically active protein fragment is separated from the hydrolysate by gel chromatography.

22. A pharmaceutical composition, **characterized by** a content of a protein according to any one of claims 17 to 20 and conventional carriers and/or diluents and/or adjuvants.

23. The pharmaceutical composition according to claim 22, as a spray or inhalant.

24. The pharmaceutical composition according to claim 22 or 23 for the treatment of chronic bronchitis, chronic cervix inflammation, of chronic inflammatory processes associated with excessive formation of mucus and the emergency situations resulting therefrom as well as of post-operative haemorrhage due to a hyperfibrinolysis and for the early therapy of shock.

**Claims for the following Contracting State : GR**

1. A DNA sequence, **characterized in that** it encodes a protein having the biological activity of the CUSI-I protein with the amino acid sequence shown in Figure 4.

2. A DNA sequence, **characterized in that** it encodes a protein having the biological activity of the CUSI-I protein with the amino acid sequence shown in Figure 5.

3. A DNA sequence, **characterized in that** it encodes a protein having the biological activity of the CUSI-I protein and is the sequence shown in Figure 4 which is contained in the recombinant plasmid pRH 31 (DSM 3634) as PstI fragment.

4. A DNA sequence, **characterized in that** it encodes a protein having the biological activity of the CUSI-I protein and is the sequence shown in Figure 5 which is contained in the recombinant plasmid pRH 34 (DSM 3635) as PstI fragment.

5. A DNA fragment with the sequence

```
5' A A T T C G G A G G T G T C G A C T A T G A A G T C C A G C G G 3'
        G C C T C C A C A G C T G A T A C T T C A G G T C G C C
```

6. A recombinant vector for cloning, **characterized in that** it contains a DNA sequence according to any one of claims 1 to 5.

7. A recombinant vector for expression, **characterized in that** it contains a DNA sequence according to any one of claims 1 to 5 which is operatively linked with an expression control sequence.

8. The recombinant vector according to claim 7, **characterized in that** the expression control sequence is the E. coli lac promoter, the E. coli trp promoter, the E. coli lipoprotein promoter, the lambda-PL or PR promoter, a yeast expression control sequence or another eukaryotic expression control sequence.

9. Plasmid pRH 31 (DSM 3634).

10. Plasmid pRH 34 (DSM 3635).

11. Plasmid pRH 1810 (DSM 3905).

12. Plasmid pRH 24, obtainable according to the construction scheme in Figure 7 starting from plasmid pRH 1810 (DSM 3905).

28

13. Plasmid pRH 21, obtainable according to the construction scheme in Figure 10 starting from plasmid pRH 31 (DSM 3634).

14. Plasmid pBA17, obtainable according to the construction scheme in Figure 11 starting from plasmid pRH 1810 (DSM 3905).

15. A host organism, **characterized in that** it is transformed with one of the recombinant vectors according to claims 6 to 14.

16. The host organism according to claim 15, **characterized in that** it is a strain of the species E. coli, B. subtilis or another bacterium, Saccharomyces cerevisiae, another microscopically small fungus, an animal or a human cell.

17. A protein, **characterized in that** it has the biological activity of the CUSI-I protein and it has the amino acid sequence shown in Figure 4.

18. A protein, **characterized in that** it has the biological activity of the CUSI-I protein and it has the amino acid sequence shown in Figure 5.

19. A protein, **characterized in that** it has the biological activity of the CUSI-I protein and it has the amino acid sequence

Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH.

20. A protein, **characterized in that** it has the biological activity of the CUSI-I protein and it has the amino acid sequence

Asp-Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH.

21. A method for the preparation of a protein according to any one of claims 17 to 20, **characterized in that** a host organism according to claim 15 or 16 is cultured in a conventional nutrient medium, the expression of the gene product is optionally induced, the expression product is isolated from the culture and optionally subjected to a controlled acidic hydrolysis and the desired biologically active protein fragment is separated from the hydrolysate by gel chromatography.

**Claims for the following Contracting States : AT, ES**

1. A method for the preparation of a DNA sequence encoding a protein having the biological activity of the CuSI-I protein with the amino acid sequence shown in Figure 4, **characterized in that** a gene coding for the synthesis of the protein is identified and isolated by means of recombinant DNA technology.

2. A method for the preparation of a DNA sequence encoding a protein having the biological activity of the CUSI-I protein with the amino acid sequence shown in Figure 5, **characterized in that** a gene coding for the synthesis of the protein is identified and isolated by means of recombinant DNA technology.

3. A method for the preparation of a DNA sequence according to claim 1, **characterized in that** the DNA sequence is contained in the recombinant plasmid pRH 31 (DSM 3634) as PstI fragment.

4. The method for the preparation of a DNA sequence according to claim 2, **characterized in that** the DNA sequence is contained in the recombinant plasmid pRH 34 (DSM 3635) as PstI fragment.

5. A method for the preparation of a DNA fragment with the sequence

$$5' \; A \; A \; T \; T \; C \; G \; G \; A \; G \; G \; T \; G \; T \; C \; G \; A \; C \; T \; A \; T \; G \; A \; A \; G \; T \; C \; C \; A \; G \; C \; G \; G \; 3'$$
$$G \; C \; C \; T \; C \; C \; A \; C \; A \; G \; C \; T \; G \; A \; T \; A \; C \; T \; T \; C \; A \; G \; G \; T \; C \; G \; C \; C$$

**characterized in that** two synthetic, complementary oligonucleotides are hybridized with one another.

6. A method for the preparation of a recombinant vector for cloning, **characterized in that** a DNA sequence prepared according to any one of the methods of claims 1 to 5 is cloned optionally after ligation according to Fig. 7 into a conventional cloning vector.

7. A method for the preparation of a recombinant vector for expression, **characterized in that** a DNA sequence prepared according to any one of the methods of claims 1 to 5 is cloned optionally after ligation according to Fig. 7 into a conventional expression vector such that the DNA sequence is operatively linked with an expression control sequence.

8. The method for the preparation of a recombinant vector according to claim 7, **characterized in that** the expression control sequence is the E. coli lac promoter, the E. coli trp promoter, the E. coli lipoprotein-promoter, the lambda-PL or PR promoter, a yeast-expression-control sequence or another eukaryotic expression control sequence.

9. A method for the preparation of the plasmid pRH 24, **characterized by** proceeding according to the construction scheme of Fig. 7 starting from the plasmid pRH 1810 (DSM 3905).

10. A method for the preparation of the plasmid pRH 21, **characterized by** proceeding according to the construction scheme of Fig. 10 starting from the plasmid pRH 31 (DSM 3634).

11. A method for the preparation of the plasmid pBA 17, **characterized by** proceeding according to the construction scheme of Fig. 11 starting from the plasmid pRH 1810 (DSM 3905).

12. A method for the preparation of a transformed host organism, **characterized in that** a host organism is transformed with one of the vectors prepared according to any one of methods 6 to 11.

13. The method for the preparation of a transformed host organism according to claim 12, **characterized in that** the host organism is a strain of the species E. coli, B. subtilis or another bacterium, Saccharomyces cerevisiae, another microscopically small fungus, an animal or a human cell.

14. A method for the preparation of a protein having the biological activity of the CUSI-I protein and having the amino acid sequence shown in Fig. 4, **characterized in that** a host organism preparable according to any one of the methods of claim 12 or 13 is cultured in a conventional nutrient medium, the expression of the gene product is optionally induced, the expression product is isolated from the culture and optionally subjected to a controlled acidic hydrolysis and the desired biologically active protein fragment is separated from the hydrolysate by gel chromatography.

15. A method for the preparation of a protein having the biological activity of the CUSI-I protein and having the amino acid sequence shown in Fig. 5, **characterized by** proceeding as in claim 14.

16. The method for the preparation of a protein according to claim 14 which comprises a partial region of the amino acid sequence shown in Fig. 4, **characterized in that** the protein has the amino acid sequence

Pro-Val-Asp-Thr-Pro-Asn-Pro-Thr-Arg-Arg-Lys-Pro-Gly-Lys-Cys-Pro-Val-Thr-Tyr-Gly-Gln-Cys-Leu-Met-Leu-Asn-Pro-Pro-Asn-Phe-Cys-Glu-Met-Asp-Gly-Gln-Cys-Lys-Arg-Asp-Leu-Lys-Cys-Cys-Met-Gly-Met-Cys-Gly-Lys-Ser-Cys-Val-Ser-Pro-Val-Lys-Ala-OH

17. The method for the preparation of a protein according to claim 14 which comprises a partial region of the amino acid sequence shown in Fig. 4, **characterized in that** the protein has the amino acid sequence

Asp-Pro-Val-Asp-Thr-Pro-Asn-Pro-Thr-Arg-Arg-Lys-Pro-Gly-Lys-Cys-Pro-Val-Thr-Tyr-Gly-Gln-Cys-Leu-Met-Leu-Asn-Pro-Pro-Asn-Phe-Cys-Glu-Met-Asp-Gly-Gln-Cys-Lys-Arg-Asp-Leu-Lys-Cys-Cys-Met-Gly-Met-Cys-Gly-Lys-Ser-Cys-Val-Ser-Pro-Val-Lys-Ala-OH.

18. A method for the preparation of a pharmaceutical composition, **characterized in that** a protein prepared according to any one of the methods of claims 14 to 17 is mixed with conventional carriers and/or diluents and/or adjuvants.

19. The method for the preparation of a pharmaceutical composition according to claim 18, **characterized in that** the pharmaceutical composition can be used as a spray or an inhalant.

20. The method for the preparation of a pharmaceutical composition according to claim 18 or 19, **characterized in that** the pharmaceutical composition can be used for the treatment of chronic bronchitis, chronic cervix inflammation, of chronic inflammatory processes associated with excessive formation of mucus and the emergency situations resulting therefrom as well as of post-operative haemorrhage due to a hyperfibrinolysis and for the early therapy of shock.

**Revendications**
**Revendications pour les Etats contractants suivants : BE, CH, DE, FR, GB, IT, LI, LU, NL, SE**

1. Séquence d'ADN, caractérisée en ce qu'elle code une protéine ayant l'activité biologique de la protéine CUSI-I ayant la séquence d'acides aminés représentée à la Fig. 4.

2. Séquence d'ADN, caractérisée en ce qu'elle code une protéine ayant l'activité biologique de la protéine CUSI-I ayant la séquence d'acides aminés représentée à la Fig. 5.

3. Séquence d'ADN, caractérisée en ce qu'elle code une protéine ayant l'activité biologique de la protéine CUSI-I et est la séquence représentée à la Fig. 4 qui est contenue à l'état de fragment Pst-I dans le plasmide recombinant pRH 31 (DSM 3634).

4. Séquence d'ADN, caractérisée en ce qu'elle code une protéine ayant l'activité biologique de la protéine CUSI-I et est la séquence représentée à la Fig. 5 qui est contenue à l'état de fragment Pst-I dans le plasmide recombinant pRH 34 (DSM 3635).

5. Fragment d'ADN ayant la séquence

5' A A T T C G G A G G T G T C G A C T A T G A A G T C C A G C G G 3'
    G C C T C C A C A G C T G A T A C T T C A G G T C G C C

6. Vecteur recombinant pour le clonage, caractérisé en ce qu'il contient une séquence d'ADN suivant l'une quelconque des revendications 1 à 5.

7. Vecteur recombinant pour l'expression, caractérisé en ce qu'il contient une séquence d'ADN suivant l'une quelconque des revendications 1 à 5 qui est unie fonctionnellement à une séquence de contrôle d'expression.

8. Vecteur recombinant suivant la revendication 7, caractérisé en ce que la séquence de contrôle d'expression est le promoteur lac de E. coli, le promoteur trp de E. coli, le promoteur lipoprotéine de E. coli, le promoteur lambda-PL ou -PR, une séquence de contrôle d'expression de levure ou une autre séquence d'expression d'eucaryote.

9. Le plasmide pRH 31 (DSM 3634).

10. Le plasmide pRH 34 (DSM 3635).

11. Le plasmide pRH 1810 (DSM 3905).

12. Le plasmide pRH 24, s'obtenant conformément au schéma de construction suivant la Fig. 7 à partir du plasmide pRH 1810 (DSM 3905).

13. Le plasmide pRH 21, s'obtenant conformément au schéma de construction suivant la Fig. 10 à partir du plasmide pRH 31 (DSM 3634).

14. Le plasmide pBA17, s'obtenant conformément au schéma de construction suivant la Fig. 11 à partir du plasmide pRH 1810 (DSM 3905).

15. Organisme hôte, caractérisé en ce qu'il est transformé avec l'un des vecteurs recombinants suivant les revendications 6 à 14.

16. Organisme hôte suivant la revendication 15, caractérisé en ce qu'il est une souche de l'espèce E. coli, B. subtilis ou une autre bactérie, Saccharomyces cerevisiae, un autre petit champignon microscopique, une cellule animale ou une cellule humaine.

17. Protéine, caractérisée en ce qu'elle manifeste l'activité biologique de la protéine CUSI-I et a la séquence d'acides aminés représentée à la Fig. 4.

18. Protéine, caractérisée en ce qu'elle manifeste l'activité biologique de la protéine CUSI-I et a la séquence d'acides aminés représentée à la Fig. 5.

19. Protéine, caractérisée en ce qu'elle manifeste l'activité biologique de la protéine CUSI-I et a la séquence d'acides aminés

```
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH
```

20. Protéine, caractérisée en ce qu'elle manifeste l'activité biologique de la protéine CUSI-I et a la séquence d'acides aminés

```
Asp-Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH.
```

21. Procédé de préparation d'une protéine suivant l'une quelconque des revendications 17 à 20, caractérisé en ce qu'on cultive un organisme hôte suivant la revendication 15 ou 16 dans un milieu nutritif habituel, on induit éventuellement l'expression du produit génique, on isole le produit d'expression hors de la culture et on le soumet éventuellement à une hydrolyse acide contrôlée et on sépare le fragment de protéine biologiquement actif souhaité hors de l'hydrolysat par chromatographie sur gel.

22. Médicament, caractérisé en ce qu'il contient une protéine suivant l'une quelconque des revendications 17 à 20 et des excipients et/ou diluants et/ou adjuvants habituels.

23. Médicament suivant la revendication 22, à l'état de spray ou d'agent pour inhalation.

24. Médicament suivant la revendication 22 ou 23 pour le traitement de la bronchite chronique, des inflammations chroniques du col, des processus inflammatoires chroniques associés à une formation excessive de mucus et des situations de détresse qui en résultent, de même que des hémorragies postopératoires dues à une hyperfibrinolyse, et pour le traitement précoce des états de choc.

**Revendications pour l'Etat contractant suivant : GR**

1. Séquence d'ADN, caractérisée en ce qu'elle code une protéine ayant l'activité biologique de la protéine CUSI-I ayant la séquence d'acides aminés représentée à la Fig. 4.

2. Séquence d'ADN, caractérisée en ce qu'elle code une protéine ayant l'activité biologique de la protéine CUSI-I ayant la séquence d'acides aminés représentée à la Fig. 5.

3. Séquence d'ADN, caractérisée en ce qu'elle code une protéine ayant l'activité biologique de la protéine CUSI-I et est la séquence représentée à la Fig. 4 qui est contenue à l'état de fragment Pst-I dans le plasmide recombinant pRH 31 (DSM 3634).

4. Séquence d'ADN, caractérisée en ce qu'elle code une protéine ayant l'activité biologique de la protéine CUSI-I et est la séquence représentée à la Fig. 5 qui est contenue à l'état de fragment Pst-I dans le plasmide recombinant pRH 34 (DSM 3635).

5. Fragment d'ADN ayant la séquence

```
5' A A T T C G G A G G T G T C G A C T A T G A A G T C C A G C G G 3'
      G C C T C C A C A G C T G A T A C T T C A G G T C G C C
```

6. Vecteur recombinant pour le clonage, caractérisé en ce qu'il contient une séquence d'ADN suivant l'une quelconque des revendications 1 à 5.

7. Vecteur recombinant pour l'expression, caractérisé en ce qu'il contient une séquence d'ADN suivant l'une quelconque des revendications 1 à 5 qui est unie fonctionnellement à une séquence de contrôle d'expression.

8. Vecteur recombinant suivant la revendication 7, caractérisé en ce que la séquence de contrôle d'expression est le promoteur lac de E. coli, le promoteur trp de E. coli, le promoteur lipoprotéine de E. coli, le promoteur lambda-PL ou -PR, une séquence de contrôle d'expression de levure ou une autre séquence d'expression d'eucaryote.

9. Le plasmide pRH 31 (DSM 3634).

10. Le plasmide pRH 34 (DSM 3635).

11. Le plasmide pRH 1810 (DSM 3905).

12. Le plasmide pRH 24, s'obtenant conformément au schéma de construction suivant la Fig. 7 à partir du plasmide pRH 1810 (DSM 3905).

13. Le plasmide pRH 21, s'obtenant conformément au schéma de construction suivant la Fig. 10 à partir du plasmide pRH 31 (DSM 3634).

14. Le plasmide pBA17, s'obtenant conformément au schéma de construction suivant la Fig. 11 à partir du plasmide pRH 1810 (DSM 3905).

15. Organisme hôte, caractérisé en ce qu'il est transformé avec l'un des vecteurs recombinants suivant les revendications 6 à 14.

16. Organisme hôte suivant la revendication 15, caractérisé en ce qu'il est une souche de l'espèce E. coli, B. subtilis ou une autre bactérie, Saccharomyces cerevisiae, un autre petit champignon microscopique, une cellule animale ou une cellule humaine.

17. Protéine, caractérisée en ce qu'elle manifeste l'activité biologique de la protéine CUSI-I et a la séquence d'acides aminés représentée à la Fig. 4.

18. Protéine, caractérisée en ce qu'elle manifeste l'activité biologique de la protéine CUSI-I et a la séquence d'acides aminés représentée à la Fig. 5.

19. Protéine, caractérisée en ce qu'elle manifeste l'activité biologique de la protéine CUSI-I et a la séquence d'acides aminés

```
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH
```

20. Protéine, caractérisée en ce qu'elle manifeste l'activité biologique de la protéine CUSI-I et a la séquence d'acides aminés

```
Asp-Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Met-Leu-Asn-Pro-Pro-Asn-Phe-Cys-Glu-Met-Asp-
Gly-Gln-Cys-Lys-Arg-Asp-Leu-Lys-Cys-Cys-Met-Gly-Met-Cys-Gly-Lys-Ser-
Cys-Val-Ser-Pro-Val-Lys-Ala-OH
```

21. Procédé de préparation d'une protéine suivant l'une quelconque des revendications 17 à 20, caractérisé en ce qu'on cultive un organisme hôte suivant la revendication 15 ou 16 dans un milieu nutritif habituel, on induit éventuellement l'expression du produit génique, on isole le produit d'expression hors de la culture et on le soumet éventuellement à une hydrolyse acide contrôlée et on sépare le fragment de protéine biologiquement actif souhaité hors de l'hydrolysat par chromatographie sur gel.

**Revendications pour les Etats contractants suivants : AT, ES**

1. Procédé de préparation d'une séquence d'ADN qui code une protéine ayant l'activité biologique de la protéine CUSI ayant la séquence d'acides aminés représentée à la Fig. 4, caractérisé en ce qu'un gène codant pour la synthèse de la protéine est identifié et isolé suivant la technologie de l'ADN recombinant.

2. Procédé de préparation d'une séquence d'ADN qui code une protéine ayant l'activité biologique de la protéine CUSI ayant la séquence d'acides aminés représentée à la Fig. 5, caractérisé en ce qu'un gène codant pour la synthèse de la protéine est identifié et isolé suivant la technologie de l'ADN recombinant.

3. Procédé de préparation d'une séquence d'ADN suivant la revendication 1, caractérisé en ce que la séquence d'ADN est contenue à l'état de fragment Pst-I dans le plasmide recombinant pRH 31 (DSM 3634).

4. Procédé de préparation d'une séquence d'ADN suivant la revendication 2, caractérisé en ce que la séquence d'ADN est contenue à l'état de fragment Pst-I dans le plasmide recombinant pRH 34 (DSM 3635).

5. Procédé de préparation d'un fragment d'ADN ayant la séquence

```
5' A A T T C G G A G G T G T C G A C T A T G A A G T C C A G C G G 3'
      G C C T C C A C A G C T G A T A C T T C A G G T C G C C
```

caractérisé en ce que deux oligonucléotides synthétiques mutuellement complémentaires sont hybridés l'un à l'autre.

6. Procédé de préparation d'un vecteur recombinant pour le clonage, caractérisé en ce qu'une séquence d'ADN préparée par l'un des procédés suivant les revendications 1 à 5 éventuellement après ligature suivant la Fig. 7 est clonée dans un vecteur de clonage habituel.

7. Procédé de préparation d'un vecteur recombinant pour l'expression, caractérisé en ce qu'une séquence d'ADN préparée par l'un des procédés suivant les revendications 1 à 5 éventuellement après ligature suivant la Fig. 7 est clonée dans un vecteur d'expression habituel de façon que la séquence d'ADN soit unie fonctionnellement avec une séquence de contrôle d'expression.

8. Procédé de préparation d'un vecteur recombinant suivant la revendication 7, caractérisé en ce que la séquence de contrôle d'expression est le promoteur lac de E. coli, le promoteur trp de E. coli, le promoteur lipoprotéine de E. coli, le promoteur lambda-PL ou -PR, une séquence de contrôle d'expression de levure ou une autre séquence d'expression d'eucaryote.

9. Procédé de préparation du plasmide pRH 24, caractérisé en ce qu'on procède conformément au schéma de construction suivant la Fig. 7 à partir du plasmide pRH 1810 (DSM 3905).

10. Procédé de préparation du plasmide pRH 21, caractérisé en ce qu'on procède conformément au schéma de construction suivant la Fig. 10 à partir du plasmide pRH 31 (DSM 3634).

11. Procédé de préparation du plasmide pBa 17, caractérisé en ce qu'on procède conformément au schéma de construction suivant la Fig. 11 à partir du plasmide pRH 1810 (DSM 3905).

12. Procédé de préparation d'un organisme hôte transformé, caractérisé en ce qu'un organisme hôte est transformé avec l'un des vecteurs préparés par l'un des procédés suivant les revendications 6 à 11.

13. Procédé de préparation d'un organisme hôte transformé suivant la revendication 12, caractérisé en ce que l'organisme hôte est une souche de l'espèce E. coli, B. subtilis ou une autre bactérie, Saccharomyces cerevisiae, un autre petit champignon microscopique, une cellule animale ou une cellule humaine.

14. Procédé de préparation d'une protéine qui manifeste l'activité biologique de la protéine CUSI-I et a la séquence d'acides aminés représentée à la Fig. 4, caractérisé en ce qu'on cultive un organisme hôte qui peut être préparé par l'un des procédés suivant la revendication 12 ou 13 dans un milieu nutritif habituel, on induit éventuellement l'expression du produit génique, on isole le produit d'expression hors du milieu de culture et on le soumet éventuellement à une hydrolyse acide contrôlée et on sépare le fragment de protéine biologiquement actif souhaité hors de l'hydrolysat par chromatographie sur gel.

15. Procédé de préparation d'une protéine qui manifeste l'activité biologique de la protéine CUSI-I et a la séquence d'acides aminés représentée à la Fig. 5, caractérisé en ce qu'on procède comme dans la revendication 14.

16. Procédé de préparation d'une protéine suivant la revendication 14, qui comprend un domaine partiel de la séquence d'acides aminés représentée à la Fig. 4, caractérisé en ce que la protéine a la séquence d'acides aminés

Pro-Val-Asp-Thr-Pro-Asn-Pro-Thr-Arg-Arg-Lys-Pro-Gly-Lys-Cys-Pro-Val-Thr-Tyr-Gly-Gln-Cys-Leu-Met-Leu-Asn-Pro-Pro-Asn-Phe-Cys-Glu-Met-Asp-Gly-Gln-Cys-Lys-Arg-Asp-Leu-Lys-Cys-Cys-Met-Gly-Met-Cys-Gly-Lys-Ser-Cys-Val-Ser-Pro-Val-Lys-Ala-OH

17. Procédé de préparation d'une protéine suivant la revendication 14, qui comprend un domaine partiel de la séquence d'acides aminés représentée à la Fig. 4, caractérisé en ce que la protéine a la séquence d'acides aminés

Asp-Pro-Val-Asp-Thr-Pro-Asn-Pro-Thr-Arg-Arg-Lys-Pro-Gly-Lys-Cys-Pro-Val-Thr-Tyr-Gly-Gln-Cys-Leu-Met-Leu-Asn-Pro-Pro-Asn-Phe-Cys-Glu-Met-Asp-Gly-Gln-Cys-Lys-Arg-Asp-Leu-Lys-Cys-Cys-Met-Gly-Met-Cys-Gly-Lys-Ser-Cys-Val-Ser-Pro-Val-Lys-Ala-OH

18. Procédé de préparation d'un médicament, caractérisé en ce qu'on mélange une protéine préparée par l'un des procédés suivant les revendications 14 à 17 avec des excipients et/ou diluants et/ou adjuvants habituels.

19. Procédé de préparation d'un médicament suivant la revendication 18, caractérisé en ce que le médicament peut être utilisé comme spray ou comme agent pour inhalation.

20. Procédé de préparation d'un médicament suivant la revendication 18 ou 19, caractérisé en ce que le médicament peut être utilisé pour le traitement de la bronchite chronique, des inflammations chroniques du col, des processus inflammatoires chroniques associés à une formation excessive de mucus et des situations de détresse qui en résultent, de même que des hémorragies postopératoires dues à une hyperfibrinolyse, et pour le traitement précoce des états de choc.

Fig. 1: Aminosäuresequenz tryptischer Fragmente von
HUSI-I

```
        1                                          10                    15
T1    Lys-Arg-Cys-Cys-Pro-Asp-Thr-Cys-Gly-Ile-Lys-Cys-Leu-Asp-Pro-
        16                              23
      Val-Asp-Thr-Pro-Asn-Pro-Thr-Arg


        1                                          10                    15
T2    Lys-Pro-Gly-Lys-Cys-Pro-Val-Thr-Tyr-Gly-Gln-Cys-Leu-Met-Leu
        16              20                                          29
      Asn-Pro-Pro-Asn-Phe-Cys-Glu-Met-Asp-Gly-Gln-Cys-Lys-Arg
                    3'    AAG ACG CTT TAC CTG CC      5'
                          A   A   C       A
                    |———————RH1———————|


        1                                        11
T3    Asp-Leu-Lys-Cys-Cys-Met-Gly-Met-Cys-Gly-Lys
                3'    ACG ACG TAC CCA TAC AC      5'
                      A   A       G
                              T
                              C
                    |—·——RH2————————|


        1                          8
T4    Ser-Cys-Val-Ser-Pro-Val-Lys-Ala
```

Fig 2: Restriktionskarte des Plasmids pRH 31

Fig. 3: Schema der Sequenzierungsstrategie der
cDNA-Insertion des Plasmids pRH 31

Fig. 4: Nucleotidsequenz des CUSI-I-cDNA-Fragmentes des
Plasmids pRH 31

```
                                          Cys   Leu   Arg   Tyr   Lys   Lys   Pro
C T G C A G G G G G G G G G G G G G G G G T G C C T T A G A T A C A A G A A A C C T      45
G A C G T C C C C C C C C C C C C C C C C A C G G A A T C T A T G T T C T T T G G A
                                                    |————————————————RH-5——————————

  Glu   Cys   Gln   Ser   Asp   Trp   Gln   Cys   Pro   Gly   Lys . Lys   Arg   Cys   Cys
G A G T G C C A G A G T G A C T G G C A G T G T C C A G G G A A G A A G A G A T G T T G T   90
C T C A C G G T C T C A C T G A C C G T C A C A G G T C C C T T C T T C T C T A C A A C A
|—————————————|

  Pro   Asp   Thr   Cys   Gly   Ile   Lys   Cys   Leu   Asp   Pro   Val   Asp   Thr   Pro
C C T G A C A C T T G T G G C A T C A A A T G C C T G G A T C C T G T T G A C A C C C C A   135
G G A C T G T G A A C A C C G T A G T T T A C G G A C C T A G G A C A A C T G T G G G G T

  Asn   Pro   Thr   Arg   Arg   Lys   Pro   Gly   Lys   Cys   Pro   Val   Thr   Tyr   Gly
A A C C C A A C A A G G A G G A A G C C T G G G A A G T G C C C A G T G A C T T A T G G C   180
T T G G G T T G T T C C T C C T T C G G A C C C T T C A C G G G T C A C T G A A T A C C G

  Gln   Cys   Leu   Met   Leu   Asn   Pro   Pro   Asn   Phe   Cys   Glu   Met   Asp   Gly
C A A T G T T T G A T G C T T A A C C C C C C C A A T T T C T G T G A G A T G G A T G G C   225
G T T A C A A A C T A C G A A T T G G G G G G G T T A A A G A C A C T C T A C C T A C C G
                                                    |—————————————— RH-1 ——————————|

  Gln   Cys   Lys   Arg   Asp   Leu   Lys   Cys   Cys   Met   Gly   Met   Cys   Gly   Lys
C A G T G C A A G C G T G A C T T G A A G T G T T G C A T G G G C A T G T G T G G G A A A   270
G T C A C G T T C G C A C T G A A C T T C A C A A C G T A C C C G T A C A C A C C C T T T
                              |—————————————— RH-2 ——————————|

  Ser   Cys   Val   Ser   Pro   Val   Lys   Ala   Stop
T C C T G C G T T T C C C C T G T G A A A G C T T G A T T C C T G C C A T A T G G A G      313
A G G A C G C A A A G G G G A C A C T T T C G A A C T A A G G A C G G T A T A C C T C
```

**358**

C G
C C
C G
A T
C G
C G
T A
T A
T A
C G
C G
T A
G C
G C
A T
C G
C G
T A
G C
G C
T A
G C
T A
G C
T A
C G
T A
C G
T A
C G
G C
T A
C G
T A
C G
A T
G C
G C
T A
C G
T A
C G
A T
G C

**403**

T A
T A
C G
G C
G C
A T
A T
A T
G C
G C
G C
T A
T A
T A
C G
C G
T A
A T
A T
A T
G C
T A
C G
A T
C G
C G
A T
C G
C G
C G
C G
T A
C G
C G
C G
T A
C G
G C
T A
G C
A T
G C
T A
T A

**448**

A T
A T
T A
A T
A T
G C
T A
A T
A T
C G
T A
A T
G C
T A
T A
G C
A T
C G
C G
G C
T A
G C
A T
A T
A T
C G
T A
C G
T A
G C
A T
A T
C G
G C
A T
C G
C G
A T
C G
A T
C G
C G
G C
G C

**493**

C G
C C
C G
C G
C G
C G
C G
C G
C G
C G
C G
C G
C G
C G
C G
C G
C G
C G
C G
A T
C G
G C
T A
T A
T A
C G
T A
T A
A T
T A
C G
A T
C G
G C
A T
C G
C G
C G
A G
A T
A T
T A

A G
A T
C G
G C
T A

Fig. 5: Nucleotidsequenz des CUSI-I-cDNA-Fragmentes des
Plasmids pRH 34

C T G C A G G G G G G G G G G G G G G G G G G G G G G G G G G G G G G G G G G G G G G T C A C T    46
G A C G T C C C C C C C C C C C C C C C C C C C C C C C C C C C C C C C C C C C C A G T G A

```
                        Met  Lys  Ser  Ser  Gly  Leu  Phe  Pro  Phe  Leu  Val
   C C T G C C T T C A C C A T G A A G T C C A G C G G C C T C T T C C C C T T C C T G G T G    91
   G G A C G G A A G T G G T A C T T C A G G T C G C C G G A G A A G G G G A A G G A C C A C
```

```
   Leu  Leu  Ala  Leu  Gly  Thr  Leu  Ala  Pro  Trp  Ala  Val  Glu  Gly  Ser
   C T G C T T G C C C T A G G A A C T C T G G C A C C T T G G G C T G T G G A A G G C T C T    136
   G A C G A A C G G G A T C C T T G A G A C C G T G G A A C C C G A C A C C T T C C G A G A
```

```
   Gly  Lys  Ser  Phe  Lys  Ala  Gly  Val  Cys  Pro  Pro  Lys  Lys  Ser  Ala
   G G A A A G T C C T T C A A A G C T G G A G T C T G T C C T C C T A A G A A A T C T G C C    181
   C C T T T C A G G A A G T T T C G A C C T C A G A C A G G A G G A T T C T T T A G A C G G
```

```
   Gln  Cys  Leu  Arg  Tyr  Lys  Lys  Pro  Glu  Cys  Gln  Ser  Asp  Trp  Gln
   C A G T G C C T T A G A T A C A A G A A A C C T G A G T G C C A G A G T G A C T G G C A G    226
   G T C A C G G A A T C T A T G T T C T T T G G A C T C A C G G T C T C A C T G A C C G T C
```

```
   Cys  Pro  Gly  Lys  Lys  Arg  Cys  Cys  Pro  Asp  Thr  Cys  Gly  Ile  Lys
   T G T C C A G G G A A G A A G A G A T G T T G T C C T G A C A C T T G T G G C A T C A A A    271
   A C A G G T C C C T T C T T C T C T A C A A C A G G A C T G T G A A C A C C G T A G T T T
```

```
   Cys  Leu  Asp  Pro  Val  Asp  Thr  Pro  Asn  Pro  Thr  Arg  Arg  Lys  Pro
   T G C C T G G A T C C T G T T G A C A C C C C A A A C C C A A C A A G G A G G A A G C C T    316
   A C G G A C C T A G G A C A A C T G T G G G G T T T G G G T T G T T C C T C C T T C G G A
```

EP 0 232 523 B1

EP 0 232 523 B1

```
     Gly   Lys   Cys   Pro   Val   Thr   Tyr   Gly   Gln   Cys   Leu   Met   Leu   Asn   Pro
     G G G A A G T G C C C A G T G A C T T A T G G C C A A T G T T T G A T G C T T A A C C C C     361
     C C C T T C A C G G G T C A C T G A A T A C C G G T T A C A A A C T A C G A A T T G G G G


     Pro   Asn   Phe   Cys   Glu   Met   Asp   Gly   Gln   Cys   Lys   Arg   Asp   Leu   Lys
     C C C A A T T T C T G T G A G A T G G A T G G C C A G T G C A A G C G T G A C T T G A A G     406
     G G G T T A A A G A C A C T C T A C C T A C C G G T C A C G T T C G C A C T G A A C T T C


     Cys   Cys   Met   Gly   Met   Cys   Gly   Lys   Ser   Cys   Val   Ser   Pro   Val   Lys
     T G T T G C A T G G G C A T G T G T G G G A A A T C C T G C G T T T C C C C T G T G A A A     451
     A C A A C G T A C C C G T A C A C A C C C T T T A G G A C G C A A A G G G G A C A C T T T


     Ala   Stop
     G C T T G A T T C C T G C C A T A T G G A G G A G G C T C T G G A G T C C T G C T C T G T     496
     C G A A C T A A G G A C G G T A T A C C T C C T C C G A G A C C T C A G G A C G A G A C A


     G T G G T C C A G G T C C T T T C C A C C C T G A G A C T T G G C T C C A C C C C C C C C     541
     C A C C A G G T C C A G G A A A G G T G G G A C T C T G A A C C G A G G T G G G G G G G G


     C C C C C C C C C C C C C C C C C C C T G C A G
     G G G G G G G G G G G G G G G G G G G A C G T C
```

Fig. 6: Sequenzierungsstrategie der PstI-Insertion von
pRH 1807

Fig. 7: Konstruktionsschema des Expressionsvektors pRH 24

Fig. 8: Restriktionskarte des Plasmids pRH 34

Fig. 9: Homologiebetrachtung zur intragenen Duplikation
des CUSI-I-Inhibitors

1
Ser-Gly-Lys-Ser-Phe-Lys Ala Gly Val Cys Pro Pro-Lys-Lys-Ser-Ala Gln Cys Leu Arg-Tyr-Lys-Lys Pro Glu-   (10, 20, 25)

55
Asn-Pro-Thr-Arg-Arg Lys Pro Gly Lys Cys Pro —— Val-Thr-Tyr-Gly Gln Cys Leu Met-Leu-Asn-Pro Pro Asn-   (60, 70, 78)

—— Cys Gln Ser Asp Trp Gln Cys Pro-Gly-Lys-Lys-Arg Cys Cys Pro-Asp-Thr Cys Gly Ile-Lys Cys Leu-Asp Pro Val Asp-Thr-Pro-   (30, 40, 50, 54)

79
Phe Cys Glu Met Asp Gly Gln Cys Lys-Arg-Asp-Leu-Lys Cys Cys Met-Gly-Met Cys Gly Lys-Ser Cys Val-Ser Pro Val Lys-Ala.   (90, 100, 107)

Fig. 10: Clonierungsstrategie zur Expression von CUSI-I-
Partialsequenzen als ß-Galactosidase-Fusionsprotein

Fig. 11: Konstruktionsschema des Expressionsplasmids pBA 17 zur Expression der C-terminalen CUSI-I-Domäne (= CUSI - I $2^{nd}$ domain).